(19) Europäisches Patentamt
European Patent Office
Office européen des brevets

(11) EP 0 869 121 B1

(12) # EUROPÄISCHE PATENTSCHRIFT

(45) Veröffentlichungstag und Bekanntmachung des Hinweises auf die Patenterteilung:
**09.06.2004 Patentblatt 2004/24**

(21) Anmeldenummer: **98105256.6**

(22) Anmeldetag: **24.03.1998**

(51) Int Cl.[7]: **C07D 213/74**, A61K 31/44, C07D 213/38, C07D 409/12, C07D 213/80, C07D 401/12, C07D 417/12, C07D 411/12

(54) **Hypolipidämische Propanolaminderivate**

Hypolipidemic propanol amine derivates

Dérivés de la propanolamine hypolipidémiques

(84) Benannte Vertragsstaaten:
**AT BE CH DE DK ES FI FR GB GR IE IT LI LU NL PT SE**

(30) Priorität: **04.04.1997 DE 19713865**
**26.01.1998 DE 19802530**

(43) Veröffentlichungstag der Anmeldung:
**07.10.1998 Patentblatt 1998/41**

(73) Patentinhaber: **Aventis Pharma Deutschland GmbH**
**65929 Frankfurt am Main (DE)**

(72) Erfinder:
- **Glombik, Heiner, Dr.**
  **65719 Hofheim (DE)**
- **Enhsen, Alfons, Dr.**
  **64572 Büttelborn (DE)**
- **Kramer, Werner, Dr. Dr.**
  **55130 Mainz-Laubenheim (DE)**
- **Baringhaus, Karl-Heinz, Dr.**
  **61200 Wölfersheim (DE)**

(56) Entgegenhaltungen:
**EP-A- 0 345 591**

- **CHEMICAL ABSTRACTS, vol. 99, no. 11, 12.September 1983 Columbus, Ohio, US; abstract no. 87735, LYAPOVA, M. ET AL: "Diastereomers with three neighboring phenyl groups. Part IX. Optical resolution and absolute configurations of 3-amino-1,2,3-triphenylpropanols" XP002069295 & DOKL. BOLG. AKAD. NAUK (1983), 36(2), 225-8 CODEN: DBANAD;ISSN: 0366-8681,**
- **Y HUANG ET AL.: "Hypolipidemic effects of alpha, beta, and gamma-alkylaminophenone analogs in rodents" EUROPEAN JOURNAL OF MEDICINAL CHEMISTRY.CHIMICA THERAPEUTICA, Bd. 31, Nr. 4, 1996, Seite 281-290 XP004040088**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft substituierte Propanolaminderivate und deren Säureadditionssalze.

Es sind bereits mehrere Wirkstoffklassen zur Behandlung von Adipositas und von Lipidstoffwechselstörungen beschrieben worden:

- polymere Adsorber, wie z.B. Cholestyramin
- Benzothiazepine (WO 93/16055)
- Gallensäuredimere und -konjugate (EP 0 489 423)
- 4-Amino-2-ureido-pyrimidin-5-carbonsäureamide (EP 0 557 879)

Der Erfindung lag die Aufgabe zugrunde, weitere Verbindungen zur Verfügung zu stellen, die eine therapeutisch verwertbare hypolipidämische Wirkung entfalten.

Die Erfindung betrifft daher Propanolaminderivate der Formel I,

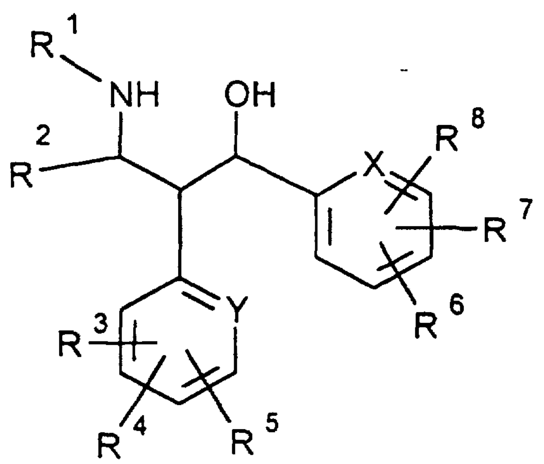

I

worin bedeuten

$R^1$ und $R^2$ unabhängig voneinander Cycloalkyl mit 3-8 Ring-C-Atomen, Phenyl, Naphthyl, Phenanthryl, Pyridyl, Thienyl, Furyl, Pyrimidyl, Indolyl, Thiazolyl, Imidazolyl, Coumarinyl, Phthaliminyl, Chinoyl, Piperazinyl, Tetrazolyl, Triazolyl, Oxazolyl oder deren thieno-, pyridino- oder benzoannelierte Derivate, wobei der Cycloalkylring, Aromat oder Heteroaromat ein bis dreifach substituiert sein kann mit Fluor, Chlor, Brom, Jod, OH, $CF_3$, $-NO_2$, CN, $(C_1-C_8)$-Alkoxy, $(C_1-C_8)$-Alkyl, $NH_2$, $-NH-R^9$, $-N(R^9)R^{10}$, CHO, -COOH, $-COOR^{11}$, $-(C{=}O)-R^{12}$, $(C_1-C_6)$-alkyl-OH, $(C_1-C_6)$-alkyl(-OH)-Phenyl, $(C_1-C_6)$-alkyl-$CF_3$, $(C_1-C_6)$-alkyl-$NO_2$, $(C_1-C_6)$-alkyl-CN, $(C_1-C_6)$-alkyl-$NH_2$, $(C_1-C_6)$-alkyl-NH-$R^9$, $(C_1-C_6)$-alkyl-$N(R^9)R^{10}$, $(C_1-C_6)$-alkyl-CHO, $(C_1-C_6)$-alkyl-COOH, $(C_1-C_6)$-alkyl-$COOR^{11}$, $(C_1-C_6)$-alkyl-$(C{=}O)-R^{12}$, -O-$(C_1-C_6)$-alkyl-OH, -O-$(C_1-C_6)$-alkyl-$CF_3$, -O-$(C_1-C_6)$-alkyl-$NO_2$, -O-$(C_1-C_6)$-alkyl-CN, -O-$(C_1-C_6)$-alkyl-$NH_2$, -O-$(C_1-C_6)$-alkyl-NH-$R^9$, -O-$(C_1-C_6)$-alkyl-$N(R^9)R^{10}$, -O-$(C_1$-$(C_1-C_6)$-alkyl-CHO, -O-$(C_1-C_6)$-alkyl-COOH, -O-$(C_1-C_6)$-alkyl-$COOR^{11}$, -O-$(C_1-C_6)$-alkyl-$(C{=}O)-R^{12}$, $-N-SO_3H$, $-SO_2-CH_3$, -O-$(C_1-C_6)$-alkyl-O-$(C_1-C_6)$-alkyl-Phenyl, wobei in den Alkylresten ein oder mehrere Wasserstoff(e) durch Fluor ersetzt sein können;

$R^3$ bis $R^8$ unabhängig voneinander Wasserstoff, Fluor, Chlor, Brom, Jod, OH, $CF_3$, $-NO_2$, CN, $(C_1-C_8)$-Alkoxy, $(C_1-C_8)$-Alkyl, $NH_2$, $-NH-R^9$, $N(R^9)R^{10}$, CHO, -COOH, $-COOR^{11}$, $-(C{=}O)-R^{12}$, wobei in den Alkylresten ein oder mehrere Wasserstoff(e) durch Fluor ersetzt sein können;

$R^9$ bis $R^{12}$ unabhängig voneinander Wasserstoff, $(C_1-C_8)$-Alkyl;

X CH, NH;

Y CH, NH;

mit der Maßgabe, daß die Reste $R^1$, $R^2$, X und Y nicht gleichzeitig die folgende Bedeutung haben

$R^1$ Phenyl, einschließlich substituiertes Phenyl;
$R^2$ Phenyl, einschließlich substituiertes Phenyl;
X CH;
Y CH;

sowie deren physiologisch verträglichen Säureadditionssalze.

Bevorzugt sind Verbindungen der Formel I, in denen ein oder mehrere Rest(e) die folgende Bedeutung hat bzw. haben:

$R^1$ und $R^2$ unabhängig voneinander Cycolalkyl mit 3-8 Ring-C-Atomen, Phenyl, Naphthyl, Thienyl, Furyl, Pyrimidyl, Thiazolyl, Imidazolyl, Phthaliminyl, Chinoyl, Piperazinyl, Tetrazolyl, Triazolyl, Oxazolyl oder deren thieno-, pyridino- oder benzoannelierte Derivate, wobei der Cycloalkylring, Aromat oder Heteroaromat ein bis dreifach substituiert sein kann mit Fluor, Chlor, Brom, OH, $CF_3$, $-NO_2$, CN, $(C_1-C_8)$-Alkoxy, $(C_1-C_8)$-Alkyl, $NH_2$, $-NH-R^9$, $-N(R^9)R^{10}$, -COOH, $-COOR^{11}$, $-(C=O)-R^{12}$, wobei in den Alkylresten ein oder mehrere Wasserstoff(e) durch Fluor ersetzt sein können;

$R^3$ bis $R^8$ unabhängig voneinander Wasserstoff, Fluor, Chlor, Brom, OH, $CF_3$, $-NO_2$, CN, $(C_1-C_8)$-Alkoxy, $(C_1-C_8)$-Alkyl, $NH_2$, $-NH-R^9$, $-N(R^9)R^{10}$, -COOH, $-COOR^{11}$, $-(C=O)-R^{12}$, wobei in den Alkylresten ein oder mehrere Wasserstoff(e) durch Fluor ersetzt sein können;

$R^9$ bis $R^{12}$ unabhängig voneinander Wasserstoff, $(C_1-C_8)$-Alkyl;

X CH, NH;

Y CH, NH; .

mit der Maßgabe, daß die Reste $R^1$, $R^2$, X und Y nicht gleichzeitig die folgende Bedeutung haben

$R^1$ Phenyl, einschließlich substituiertes Phenyl;
$R^2$ Phenyl, einschließlich substituiertes Phenyl;
X CH;
Y CH;

sowie deren physiologisch verträglichen Säureadditionssalze.

Besonders bevorzugt sind Verbindungen der Formel I, in denen ein oder mehrere Rest(e) die folgende Bedeutung hat bzw. haben:

$R^1$ Pyridyl, Pyrimidyl, Thienyl, Thiazolyl, wobei der Heteroaromat ein bis dreifach substituiert sein kann mit Fluor, Chlor, Brom, Jod, OH, $CF_3$, $-NO_2$, CN, $(C_1-C_8)$-Alkoxy, $(C_1-C_8)$-Alkyl, $NH_2$, $-NH-R^9$, $-N(R^9)R^{10}$, CHO, -COOH, $-COOR^{11}$, $-(C=O)-R^{12}$;

$R^2$ Phenyl, wobei der Aromat ein bis dreifach substituiert sein kann mit Fluor, Chlor, Brom, OH, $CF_3$, $-NO_2$, CN, $(C_1-C_8)$-Alkoxy, $(C_1-C_8)$-Alkyl, $NH_2$, $-NH-R^9$, $-N(R^9)R^{10}$, -COOH, $-COOR^{11}$, $-(C=O)-R^{12}$;

$R^3$ bis $R^8$ unabhängig voneinander Wasserstoff, Fluor, Chlor, Brom, Jod, OH, $CF_3$, $-NO_2$, CN, $(C_1-C_8)$-Alkoxy, $(C_1-C_8)$-Alkyl, $NH_2$, $-NH-R^9$, $-N(R^9)R^{10}$, CHO, -COOH, $-COOR^{11}$, $-(C=O)-R^{12}$, wobei in den Alkylresten ein oder mehrere Wasserstoff(e) durch Fluor ersetzt sein können;

$R^9$ bis $R^{12}$ unabhängig voneinander Wasserstoff, $(C_1-C_8)$-Alkyl;

X CH;

Y NH;

sowie deren physiologisch verträglichen Säureadditionssalze.

Unter physiologisch verträglichen Säureadditionssalzen werden in Wasser leicht lösliche, lösliche und wenig

lösliche Verbindungen gemäß der Definition im "Deutschen Arzneibuch" (9. Ausgabe 1986, Amtliche Ausgabe, Deutscher Apotheker-Verlag Stuttgart), Seite 19 verstanden. Bevorzugt sind die Hydrochloride und Sulfate der Verbindungen.

Gegenstand der Erfindung sind sowohl Isomerengemische der Formel I, als auch die reinen Enantiomere der Formel I.

Die Erfindung betrifft weiterhin Verfahren zur Herstellung von Propanolaminderivaten der Formel I.

Verfahren A:

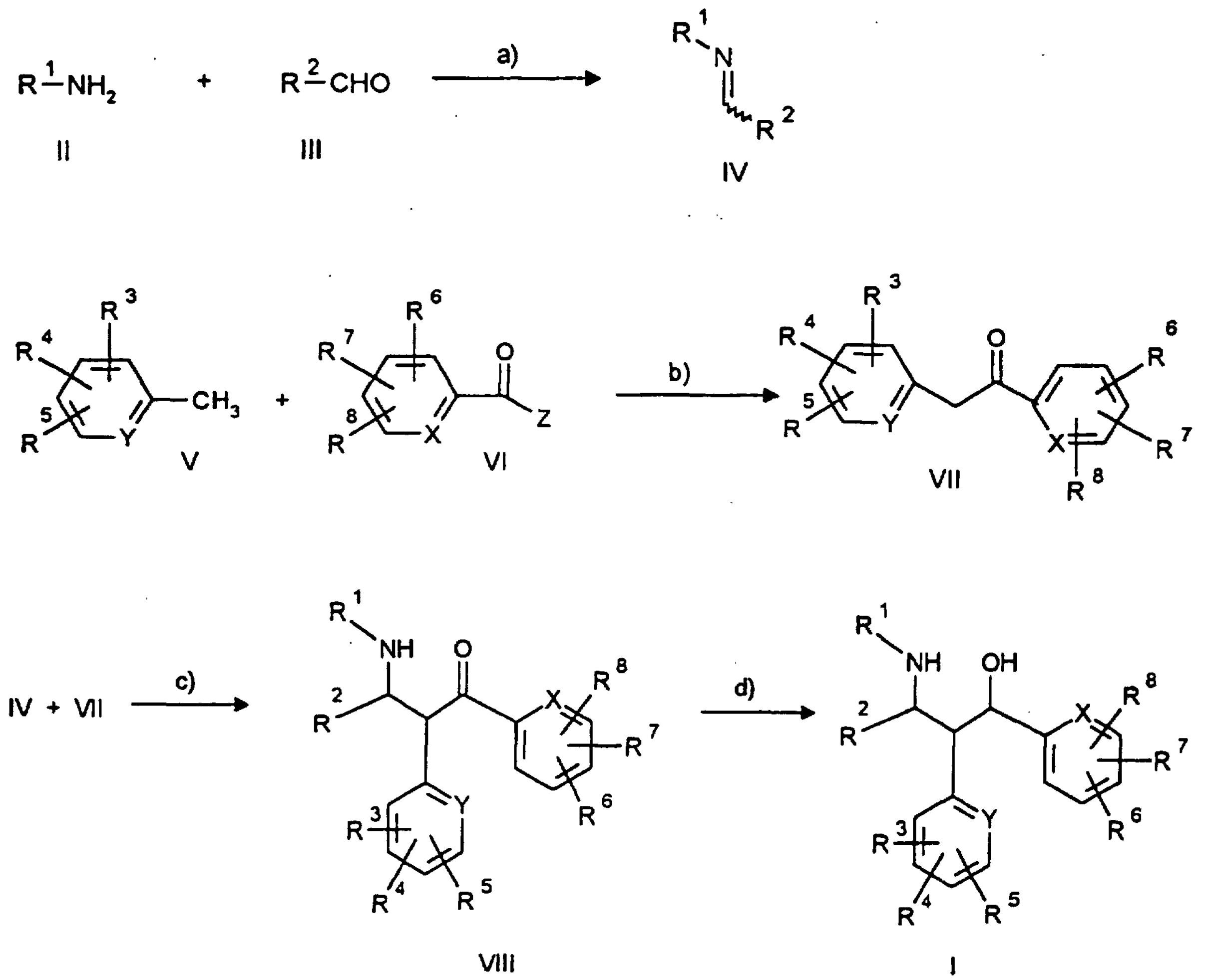

Verfahren B:

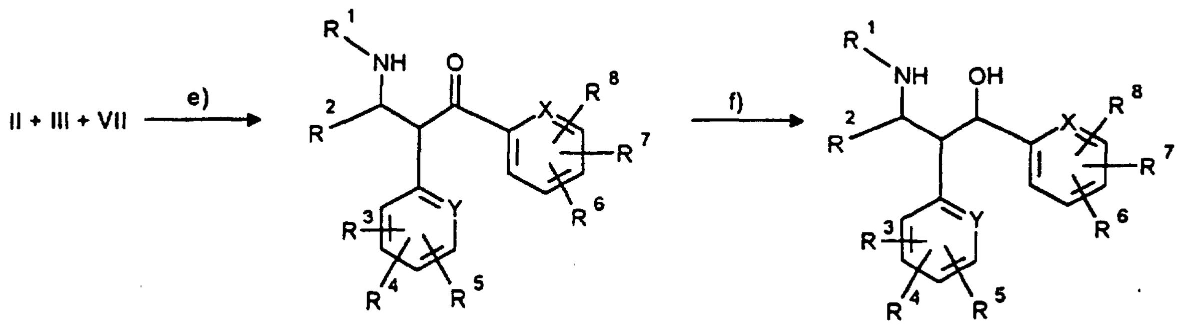


VIII I

Verfahren A zur Herstellung der Verbindungen der Formel I, dadurch gekennzeichnet, daß a) nicht literaturbekannte mit $R^1$ und $R^2$ substituierte Imine, wobei $R^1$ und $R^2$ die zu Formel I angegebene Bedeutung haben, in Anlehnung an Literaturverfahren aus Aminen des Typs II und Aldehyden des Typs III hergestellt werden. Hierzu werden zum Beispiel das Amin II und der Aldehyd III in Substanz oder in einem geeigneten Lösungsmittel wie Ethanol, Toluol oder Essigsäure ohne oder mit Zusatz einer Säure, z.B. p-Toluolsulfonsäure, bei Temperaturen von 20°-150°C zur Reaktion gebracht.

Mit Resten $R^3$ bis $R^8$ substituierte Ketoverbindungen der Formel VII, wobei $R^3$ bis $R^8$ die zu Formel I angegebene Bedeutung haben, werden nach literaturbekannten Verfahren oder in Anlehnung an solche Verfahren hergestellt. So werden beispielsweise Picolinderivate V mit geeigneten Base, wie n-Butyllithium, metalliert und in Tetrahydrofuran oder einem anderen geeigneten Lösungsmittel mit den entsprechenden Carbonsäurederivaten VI, z.B. als Carbonsäuredialkylamide oder - ester vorliegend bei Temperaturen zwischen -80° und 20°C umgesetzt.

Verbindungen des Typs VIII werden erhalten, indem Imine des Typs IV und Ketone des Typs VII, jeweils substituiert mit Resten $R^3$ bis $R^8$, wobei $R^3$ bis $R^8$ die zu Formel I angegebene Bedeutung haben, zur Reaktion gebracht werden. Diese Reaktion kann beispielsweise durch Mischung der beiden Verbindungen in Substanz, ohne Lösungsmittel und anschließendem Erhitzen, oder in einem geeigneten Lösungsmittel wie Ethanol, Toluol, Diglyme oder Tetradecan bei Temperaturen von 20°C bis 150°C durchgeführt werden (c).

Die Ketoverbindungen des Typs VIII werden in einem geeigneten Lösungsmittel, wie z.B. Methanol, THF oder THF/Wasser mit $NaBH_4$ oder einem anderen geeigneten Reduktionsmittel bei Temperaturen zwischen -30° und +40°C zu Hydroxyverbindungen des Typs I reduziert, wobei Verbindung I mit den Resten $R^3$ bis $R^8$ substituiert sein kann und $R^3$ bis $R^8$ die zu Formel I angegebene Bedeutung haben (d).

Die Verbindungen der Formel fallen nach der oben beschriebenen Reduktion als Isomerengemische an. Unterschiedliche Racemate können durch fraktionierte Kristallisation oder durch Säulenchromatographie voneinander abgetrennt werden. Die reinen Enantiomere können aus den Racematen der Verbindungen der Formel I durch Chromatographie über chirales Säulenmaterial oder durch literaturbekannte Verfahren mit optisch aktiven Hilfsreagentien, wie z.B. in J. Org. Chem. 44, 1979, 4891 beschrieben, erhalten werden.

Verfahren B zur Herstellung der Verbindungen der Formel I, dadurch gekennzeichnet, daß die Iminverbindung IV nicht wie Verfahren A hergestellt und isoliert wird, sondern die Verbindungen des Typs VIII, substituiert mit den Resten $R^3$ bis $R^8$, in einer Dreikomponentenreaktion aus Ketonen VII, Aminen II und Aldehyden III synthetisiert werden. Hierzu werden diese drei Komponenten in Substanz oder in einem geeigneten Lösungsmittel, wie Ethanol, Tetradecan oder Toluol bei Temperaturen von 20°C bis 150°C zur Reaktion gebracht (e). Die Verbindungen VIII werden, wie für Verfahren A beschrieben, zu den Verbindungen der Formel I reduziert (f), wobei die Verbindungen VIII als gereinigte Ketone, aber auch als Rohprodukte aus der oben beschriebenen Reaktion eingesetzt werden können.

Die vorliegende Erfindung betrifft auch pharmazeutische Zubereitungen, die neben nicht toxischen, inerten, pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen, inerten, pharmazeutischen geeigneten Trägerstoffen sind pharmazeutisch unbedenkliche feste, halbfeste oder flüssige Verdünnungsmittel, Füllstoffe und Formulierungshilfsmittel jeder Art zu verstehen, die nach dem Vermischen mit dem Wirkstoff diesen in eine für die Verabreichung geeignete Form bringen.

Als geeignete Anwendungsformen der erfindungsgemäßen Verbindungen kommen beispielsweise Tabletten, Dragees, Kapseln, Pillen, wäßrige Lösungen, Suspensionen und Emulsionen, ggfs. sterile injizierbare Lösungen, nichtwäßrige Emulsionen, Suspensionen und Lösungen, Sprays sowie Zubereitungsformen mit protrahierter Wirkstoff-Freigabe in Betracht.

Die therapeutisch wirksamen Verbindungen sollen in den oben aufgeführten pharmazeutischen Zubereitungen zweckmäßig in einer Konzentration von etwa 0,1 bis 99,0, vorzugsweise von 0,5 bis 70,0 Gewichtsprozenten der Gesamtmischung vorhanden sein.

Die Anwendungskonzentrationen für Lösungen sowie Aerosole in Form von Spray betragen im allgemeinen 0,1 bis 20, vorzugsweise 0,5 bis 5 Gewichtsprozent.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe(s) mit dem oder den Trägerstoff(en).

Die Wirkstoffe oder die pharmazeutischen Zubereitungen können oral, parenteral, intraperitoneal und/oder rektal appliziert werden.

Die z. B. als Hypolipidämika verwendbaren Verbindungen der vorliegenden Erfindung und ihre Salze, können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Kapseln (Gelatinekapseln), welche den Wirkstoff zusammen mit Verdünnungsmitteln bzw. Trägerstoffen, z. B. Laktose, Dextrose, Rohrzucker, Mannitol, Sorbitol, Cellulose, verschiedenen Stärkearten und/oder Glycin, und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumcarbonat, Magnesiumaluminiumsilicat, Stärke, Gelatine, Traganath, Methylcellulose, Natriumcarboxymethylcellulose und/oder Polyvinylpyrrolidon und, falls benötigt, Farbstoffe, Geschmacksstoffe und Süßmittel. Injizierbare Lösungen sind vorzugsweise isotonische wäßrige Lösungen oder Suspensionen, die sterilisiert sein können und Hilfsstoffe, wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die wenn erwünscht weitere pharmakologisch wirksame Stoffe enthalten können, werden z. B. mittels konventioneller Misch-Granulier- und Dragierverfahren, hergestellt und enthalten 0,1 % bis vorzugsweise 80 %, bevorzugt etwa 5 % bis etwa 65 %, des Wirkstoffs.

Die orale Anwendung erfolgt in pharmazeutisch üblichen Zubereitungen, beispielsweise in Form von Tabletten, Dragees oder Kapseln, die z. B. pro Tagesdosis 5 bis 1000 mg, vorzugsweise 20 bis 200 mg, des Wirkstoffes in Mischung mit einem gebräuchlichen Trägerstoff und/oder Konstituents enthalten, wobei Einzeldosen von 5 bis 200 mg, vorzugsweise ein- bis dreimal täglich, gegeben werden können.

Es kann jedoch erforderlich sein, von den genannten Dosierungen abzuweichen und zwar in Abhängigkeit von der Art und dem Körpergewicht des zu behandelnden Objekts, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die Verbindungen der Formel I und deren physiologisch verträglichen Salze stellen ideale Arzneimittel zur Behandlung von Lipidstoffwechselstörungen, insbesondere von Hyperlipidämie dar. Die Verbindungen der Formel I eignen sich ebenfalls zur Beeinflussung des Serumcholesterinspiegels sowie zur Prävention und Behandlung arteriosklerotischer Erscheinungen. Folgende Befunde belegen die pharmakologische Wirksamkeit der erfindungsgemäßen Verbindungen.

Die biologische Prüfung der erfindungsgemäßen Verbindungen erfolgte durch Ermittlung der Hemmung der [$^3$H]-Taurocholataufnahme in Bürstensaummembranvesikel des Ileums von Kaninchen. Der Hemmtest wurde wie folgt durchgeführt:

1. Präparation von Bürstensaummembranvesikeln aus dem Ileum von Kaninchen

Die Präparation von Bürstensaummembranvisikeln aus den Darmzellen des Dünndarm erfolgte mit der sogenannten $Mg^{2+}$-Präzipitationsmethode. Männliche Neuseeland-Kaninchen (2 bis 2,5 kg Körpergewicht) wurden durch intravenöse Injektion von 0,5 ml T61®, einer wäßrigen Lösung von 2,5 mg Tetracain HCl, 100 m Embutramid und 25 mg Mebezoniumjodid getötet. Der Dünndarm wurde entnommen und mit eiskalter physiologischer Kochsalzlösung gespült. Die terminalen 7/10 des Dünndarms (gemessen in oral rektaler Richtung, d.h. das terminale Ileum, welches das aktive $Na^+$-abhängige Gallensäuretransportsystem enthält) wurden zur Präparation der Bürstensaummembranvesikel verwendet. Die Därme wurden in Kunststoffbeuteln unter Stickstoff bei -80°C eingefroren. Zur Präparation der Membranvesikel wurden die eingefrorenen Därme bei 30°C im Wasserbad aufgetaut. Die Mucosa wurde abgeschabt und in 60 ml eiskaltem 12 mM Tris/HCl-Puffer (pH 7,1)/300 mM Mannit, 5 mM EGTA/10 mg/l Phenylmethyl-sulfonylfluorid/1 mg/l Trypsin Inhibitor v. Sojabohnen (32 (U/mg)/0,5 mg/l Trypsin Inhibitor v. Rinderlunge (193 U/mg)/5 mg/l Bacitracin suspendiert. Nach dem Verdünnen auf 300 ml mit eiskaltem destilliertem Wasser wurde mit einem Ultraturrax (18-Stab, IKA Werk Staufen, Deutschland) 3 Minuten bei 75 % max. Leistung unter Eiskühlung homogenisiert. Nach Zugabe von 3 ml 1 M $MgCl_2$-Lösung (Endkonzentration 10 mM) ließ man exakt 1 Minute bei 0°C stehen. Durch Zugabe von $Mg^{2+}$ aggregieren die Zellmembranen und präzipitieren mit Ausnahme der Bürstensaummembranen. Nach einer 15-minütigen Zentrifugation bei 3000 x g (5000 rpm, SS-34-Rotor) wird der Niederschlag verworfen und der Überstand, der die Bürstensaummembranen enthält, 30 Minuten bei 48000 x g (20000 rpm, SS-34-Rotor) zentrifugiert. Der Überstand wurde verworfen, der Niederschlag in 60 ml 12 mM Tris/HCl-Puffer (pH 7,1)/60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M $MgCl_2$-Lösung und 15-minütiger Inkubationszeit bei 0°C wurde erneut 15 Minuten bei 3000 x g zentrifugiert. Der Überstand wurde anschließend nochmals 30 Minuten bei 48000 x g (20000 rpm, SS-34-Rotor) zentrifugiert. Der Niederschlag wurde in 30 ml 10 mM Tris/Hepes-Puffer (pH 7,4)/300 mM Mannit aufgenommen und durch 20 Hübe in einem Potter Elvejhem Homogenisator bei 1000 rpm homogen resuspendiert. Nach 30 minütiger Zentrifugation bei 48000 x g (20000 rpm, SS-34-Rotor) wurde der Niederschlag in 0,5 bis 2 ml Tris/Hepes-Puffer (pH 7,4)/280 mM Mannit (Endkonzentration 20 mg/ml) aufgenommen und mit Hilfe einer Tuberkulinspritze mit einer 27 Gauge-Nadel resuspendiert. Die Vesikel wurden entweder unmittelbar nach der Präparation für Transportuntersuchungen verwendet oder bei -196°C in 4 mg Portionen in flüssigem Stickstoff aufbewahrt.

2. Hemmung der $Na^+$-abhängigen [$^3$H]Taurocholat-Aufnahme in Bürstensaummembranvesikel des Ileums

Die Aufnahme von Substraten in die vorstehend beschriebenen Bürstensaummembranvesikel wurde mittels der sogenannten Membranfiltrationstechnik bestimmt. 10 µl der Vesikelsuspension (100 µg Protein) wurden als Tropfen an die Wand eines Polystyrolinkubationsröhrchens (11 x 70 mm) pipettiert, welches das Inkubationsmedium mit den entsprechenden Liganden enthielt (90 µl). Das Inkubationsmedium enthielt 0,75 µl = 0,75 µCi [$^3$H(G)]-Taurocholat (spezifische Aktivität: 2,1 Ci/mMol), /0,5 µl 10 mM Taurocholat/8,75 µl Natrium-Transport-Puffer (10 mM Tris/Hepes, (pH 7,4)/100 mM Mannit/100 mM NaCl) (Na-T-P) bzw. 8,75 µl Kalium-Transport-Puffer (10 mM Tris/Hepes (pH 7,4)/ 100 mM Mannit/100 mM KCl) (K-T-P) und 80 µl der betreffenden Inhibitorlösung, je nach Experiment in Na-T-Puffer bzw. K-T-Puffer gelöst. Das Inkubationsmedium wurde durch ein Polyvinylidenfluorid-Membranfilter (SYHV LO 4NS, 0,45 µm, 4 mm ∅, Millipore, Eschborn, Deutschland) filtriert. Durch Vermischung der Vesikel mit dem Inkubationsmedium wurde die Transportmessung gestartet. Die Konzentration an Taurocholat im Inkubationsansatz betrug 50 µM. Nach der gewünschten Inkubationszeit (üblicherweise 1 Minute) wurde der Transport durch Zugabe von 1 ml eiskalter Stoplösung (10 mM Tris/Hepes, (pH 7,4)/150 mM KCl) gestoppt. Die entstehende Mischung wurde sofort bei einem Vakuum von 25 bis 35 mbar über ein Membranfilter aus Cellulosenitrat (ME 25, 0,45 µm, 25 mm Durchmesser, Schleicher & Schuell, Dassell, Deutschland) abgesaugt. Der Filter wurde mit 5 ml eiskalter Stoplösung nachgewaschen.

Zur Messung der Aufnahme des radioaktiv markierten Taurocholats wurde das Membranfilter mit 4 ml des Szintillators Quickszint 361 (Zinsser Analytik GmbH, Frankfurt, Deutschland) aufgelöst und die Radioaktivität durch Flüssigkeits-Szintillationsmessung in einem Meßgerät TriCarb 2500 (Canberra Packard GmbH, Frankfurt, Deutschland) gemessen. Die gemessenen Werte wurden nach Eichung des Gerätes mit Hilfe von Standardproben und nach Korrektur evtl. vorhandener Chemilumineszenz als dpm (Decompositions per Minute) erhalten.

Die Kontrollwerte wurden jeweils in Na-T-P und K-T-P ermittelt. Die Differenz zwischen der Aufnahme in Na-T-P und K-T-P ergab den $Na^+$-abhängigen Transportanteil. Als $IC_{50}$ $Na^+$ wurde diejenige Konzentration an Inhibitor bezeichnet, bei der der $Na^+$-abhängige Transportanteil um 50 % - bezogen auf die Kontrolle gehemmt war.

Die pharmakologischen Daten umfassen eine Testserie, in der die Interaktion der erfindungsgemäßen Verbindungen mit dem intestinalen Gallensäuretransportsystem im terminalen Dünndarm untersucht wurde. Die Ergebnisse sind in Tabelle 1 zusammengefaßt.

Tabelle 1 zeigt Meßwerte der Hemmung der [$^3$H]-Taurocholataufnahme in Bürsensaummembranvesikel des Ileums von Kaninchen. Angegeben sind die Quotienten aus den $IC_{50Na}$-Werten der Referenzsubstanz als Taurochenodesoxycholat (TCDC) und der jeweiligen Testsubstanz.

Tabelle 1:

| Verbindungen aus Beispiel | $\frac{IC_{50Na}\text{-TCDC } [\mu mol]}{IC_{50Na}\text{-Substanz } [\mu mol]}$ |
|---|---|
| 6 | 0,10 |

Tabelle 1: (fortgesetzt)

| Verbindungen aus Beispiel | $\frac{IC_{50Na}\text{-TCDC }[\mu mol]}{IC_{50Na}\text{-Substanz }[\mu mol]}$ |
|---|---|
| 10 | 0,36 |
| 32 | 0,29 |
| 36 | 0,22 |
| 61 | 0,20 |
| 70 | 0,27 |
| 72 | 0,28 |
| 83 | 0,22 |
| 86 | 0,24 |
| 101 | 0,23 |

Die nachfolgenden Beispiele dienen zur näheren Erläuterung der Erfindung, ohne dieselbe auf in den Beispielen beschriebene Produkte und Ausführungsformen einzuschränken.

Beispiel 1

a.

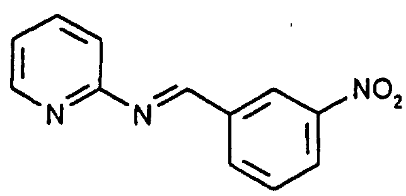

Zu einer Lösung von 25 g (266 mmol) 2-Amino-pyridin und 40 g (265 mmol) 3-Nitrobenzaldehyd in 300 ml Toluol wurden 0,7 g p-Toluolsulfonsäure zugegeben und das Gemisch 6 h unter Rückfluß erhitzt. Nach dem Abkühlen wurde die Hälfte des Lösungsmittels unter Vakuum abgezogen und über Nacht stehengelassen. Der entstandene Niederschlag wurde abgesaugt, mit kaltem Toluol gewaschen und im Vakuum getrocknet. Durch anschließende Umkristallisation aus n-Heptan/Essigsäureethylester 2:1 wurden 48,8 g (81 %) Imin erhalten.
$C_{12}H_9N_3O_2$ (227,2) MS (FAB) 228,2 M+H$^+$

b.

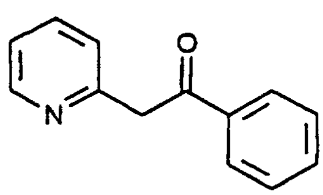

Zu einer Lösung von 50 g (0,54 mol) 2-Picolin in 770 ml Tetrahydrofuran wurden bei -55°C 250 ml n-Butyllithium (15 % in Hexan) zugetropft und 10 min gerührt. Anschließend wurde auf 0°C erwärmt und nach weiteren 30 min auf -55°C abgekühlt. Danach wurde eine Lösung von 77 g (0,52 mol) N,N-Dimethylbenzamid in 570 ml Tetrahydrofuran langsam zugetropft. Nach der Zugabe wird auf Zimmertemperatur erwärmt und 1 h gerührt. Nach der

Zugabe von 500 ml Wasser und 35 ml konz. HCl wurde die organische Phase abgetrennt und die wäßrige Phase 2 x mit Essigsäureethylester extrahiert. Nach Trocknen über $MgSO_4$ wurde im Vakuum eingeengt und der Rückstand im Hochvakuum destilliert. Siedepunkt 134-136°C/0,3 mbar. Ausbeute: 47,5 g (47 %) Keton.
$C_{13}H_{11}NO$ (197,2) MS (FAB) 198,1 M+H$^+$

c.

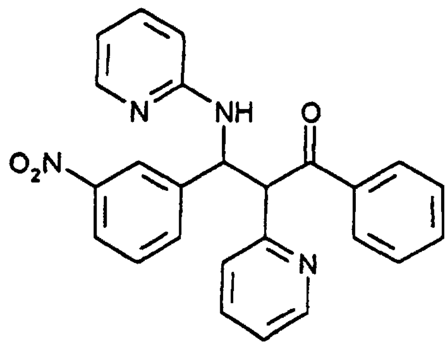

5,8 g (25,5 mmol) Imin aus Beispiel 1a und 5,0 g (25,4 mmol) Keton aus Beispiel 1 b wurden gut vermischt und auf dem Dampfbad erwärmt. Nach ca. 20 min begann die Mischung zu schmelzen und kristallisierte bei weiterer Erwärmung. Nach dem Abkühlen wurde der Rückstand in 200 ml Essigester zum Sieden erhitzt, abgekühlt, der Niederschlag abgesaugt und im Vakuum getrocknet. Ausbeute 6,7 g (62 %)
$C_{25}H_{20}N_4O_3$ (424,2) MS (FAB) 425,2 M+H$^+$

d.

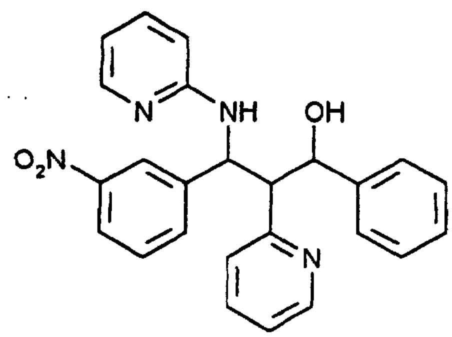

3,0 g (7,1 mmol) Ketoverbindung aus Beispiel 1c wurden in 50 ml THF/Wasser 10:1 gelöst, mit 1,35 g (35,7 mmol) Natriumborhydrid versetzt und 1 h bei Zimmertemperatur gerührt. Mit 2 N HCl wurde auf pH 1 gebracht und 30 min bei 50°C gerührt. Nach dem Abkühlen wurde das Reaktionsgemisch mit 2 N NaOH basisch gestellt und 2 x mit Essigester extrahiert. Die organischen Phasen wurden über $MgSO_4$ getrocknet und eingeengt. Der Rückstand wurde über Kieselgel mit n-Heptan/Essigester 6:4 chromatographiert. Dadurch wurden 2 racemische Verbindungen als Produkt erhalten.

1. Fraktion: 1,26 g (42 %) unpolares Racemat
$C_{25}H_{22}N_4O_3$ (426,2) MS (FAB) 427,2 M+H$^+$
2. Fraktion: 1,15 g (38 %) polares Racemat
$C_{25}H_{22}N_4O_3$ (426,2) MS (FAB) 427,2 M+H$^+$

e.

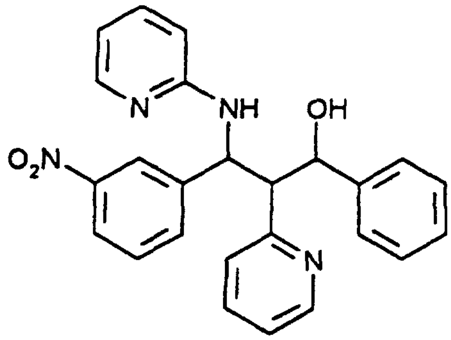

50 mg des unpolaren Racemats aus Beispiel 1 d wurden durch präparative HPLC in die Enantiomere getrennt. Die Trennung erfolgte über eine CSP Chiralpak-Säule (Fa. Daicel, Düsseldorf) mit n-Hexan/2-Propanol 50:10 + 0,1 % Diethylamin als Laufmittel. Als 1. Fraktion wurden 20 mg des (-)-Enantiomers und als 2. Fraktion 20 mg des (+)-Enantiomers erhalten.

f.

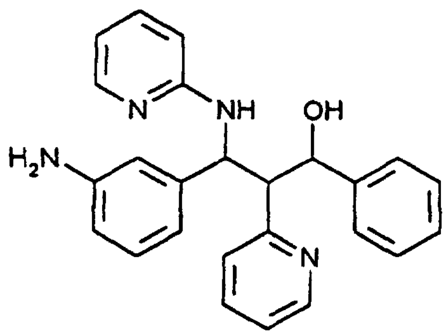

1,0 g (2,34 mmol) des unpolaren Racemats aus Beispiel 1 d wurden in 200 ml Methanol gelöst, mit ca. 20 mg Pd/C 10 % 3 h unter $H_2$-Atmosphäre bei Zimmertemperatur hydriert. Vom Katalysator wurde abfiltriert und die Lösung eingedampft. Der Rückstand wurde über Kieselgel mit Essigester/n-Heptan 4:1 chromatographiert. Ausbeute: 680 mg (73 %) Aminoverbindung $C_{25}H_{24}N_4O$ (396,2) MS (FAB) 397,3 $M+H^+$

g.
Aus 2,0 g (4,69 mmol) des polaren Racemats aus Beispiel 1 d wurden nach dem für Beispiel 1f beschriebenen Verfahren 1,2 g (65 %) der entsprechenden Aminoverbindung erhalten. $C_{25}H_{24}N_4O$ (396,2) MS (FAB) 397,2 $M+H^+$

Beispiel 2

a.

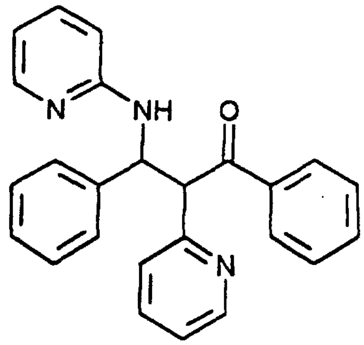

78,8 g (0,4 mol) Keton aus Beispiel 1 b, 37,6 g (0,4 mol) 2-Aminopyridin und 21,2 g (0,4 mol) Benzaldehyd wurden in 1 l Ethanol gelöst und unter gutem Rühren 1,5 h unter Rückfluß erhitzt. Danach wurde 4 h nachgerührt und über Nacht stehengelassen. Der Niederschlag wurde abgesaugt, mit wenig Ethanol gewaschen und im Vakuum getrocknet. Ausbeute 134 g (88 %).
$C_{25}H_{21}N_3O$ (379,2) MS (FAB) 380,1 $M+H^+$

b.

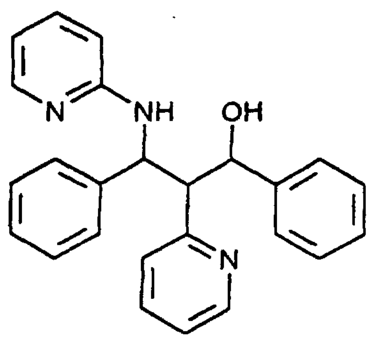

56,9 g (0,15 mol) Keton aus Beispiel 2a wurden in 1 l Methanol suspendiert und langsam portionsweise zu 60 g $NaBH_4$ in 100 ml Wasser zugegeben, Temperaturanstieg von 22°C nach 34°C. Der Alkohol wurde im Vakuum abgezogen, der Rückstand mit ca. 200 ml Wasser versetzt und 3x mit Essigester extrahiert. Die organischen Phasen wurden getrocknet und eingedampft. Der Rückstand wurde über Kieselgel mit n-Heptan/Essigester 2:1 chromatographiert. Es wurden zwei racemische Verbindungen erhalten.

1. Fraktion: 43 g (75 %) unpolares Racemat
$C_{25}H_{23}N_3O$ (381) MS (FAB) 382 $M+H^+$
2. Fraktion: 14 g (24 %) polares Racemat
$C_{25}H_{23}N_3O$ (381) MS (FAB) 382 $M+H^+$

c.

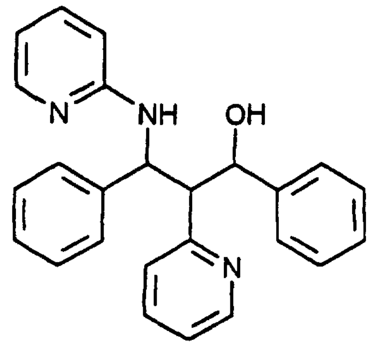

100 mg des unpolaren Racemats aus Beispiel 2b wurden nach dem unter Beispiel 1e beschriebenen Verfahren gespalten. Mit n-Hexan/2-Propanol 25:10 + 0,1 % Diethylamin als Laufmittel wurden als 1. Fraktion 40 mg des (-)-Enantiomers, als 2. Fraktion 30 mg des (+)-Enantiomers erhalten.

Beispiel 4

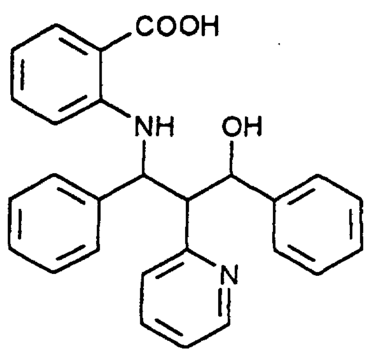

Das unpolare Racemat Beispiel 48 in Tabelle 2 wurde analog zu Beispiel 2 hergestellt, 160 mg (0,36 mmol) dieses Methylesters wurden in 20 ml Ethanol gelöst, mit 1,6 ml 2 N wäßriger NaOH-Lösung versetzt und 40 h bei Zimmertemperatur gerührt. Danach wurde das Lösungsmittel vollständig abgezogen, der Rückstand in Wasser gelöst und die Lösung mit 2 N Salzsäure auf pH 6,5 eingestellt. Es wurde 2x mit 50 ml Essigester extrahiert, die organischen Phasen getrocknet und eingeengt. Chromatographie des Rückstands über Kieselgel mit n-Heptan/Essigester 1:1 ergab 110 mg (71 %) Produkt.
$C_{27}H_{24}N_2O_3$ (424,2) FAB 425,2 M+H$^+$

Ausgehend von den entsprechenden Ausgangsverbindungen wurden die Beispiele der Tabellen 1 bis 5 analog zu den für die Beispiele 1 bis 4 beschriebenen Verfahren erhalten.

Tabelle 1

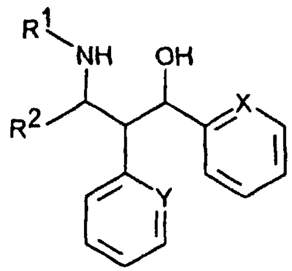

| Beispiel | $R^1$ | $R^2$ | X | Y | | Summenformel (Molmasse) | MS (FAB) |
|---|---|---|---|---|---|---|---|
| 5 | Phenyl | Phenyl | CH | N | unp. Rac. | $C_{26}H_{24}N_2O$ (380,2) | 381 $M+H^+$ |
| 6 | Phenyl | Phenyl | CH | N | pol. Rac. | $C_{26}H_{24}N_2O$ (380,2) | 381 $M+H^+$ |
| 7 | 2-Pyridyl | Phenyl | CH | CH | unp. Rac. | $C_{26}H_{24}N_2O$ (380,2) | 381 $M+H^+$ |
| 8 | 3-Pyridyl | Phenyl | CH | N | unp. Rac. | $C_{25}H_{23}N_3O$ (381,2) | 382 $M+H^+$ |
| 9 | 3-Pyridyl | Phenyl | CH | N | pol. Rac. | $C_{25}H_{23}N_3O$ (381,2) | 382 $M+H^+$ |
| 10 | 2-Pyridyl | 3-Thienyl | CH | N | pol. Rac. | $C_{23}H_{21}N_3OS$ (387) | 388 $M+H^+$ |
| 11 | 2-Pyrimidyl | Phenyl | CH | N | unp. Rac | $C_{24}H_{22}N_4O$ (382,2) | 383 $M+H^+$ |
| 12 | 2-Pyrimidyl | Phenyl | CH | N | pol. Rac. | $C_{24}H_{22}N_4O$ (382,2) | 383 $M+H^+$ |
| 13 | 2-Pyridyl | 2-Pyridyl | CH | N | unp. Rac. | $C_{24}H_{22}N_4O$ (382,2) | 383 $M+H^+$ |

| Beispiel | $R^1$ | $R^2$ | X | Y | | Summenformel (Molmasse) | MS (FAB) |
|---|---|---|---|---|---|---|---|
| 14 | 2-Pyridyl | 2-Pyridyl | CH | N | pol. Rac. | $C_{24}H_{22}N_4O$ (382,2) | 383 $M+H^+$ |
| 15 | 2-Pyridyl | 3-Pyridyl | CH | N | unp. Rac. | $C_{24}H_{22}N_4O$ (382,2) | 383 $M+H^+$ |
| 16 | 2-Pyridyl | Phenyl | N | N | unp. Rac. | $C_{24}H_{22}N_4O$ (382,2) | 383 $M+H^+$ |
| 17 | 2-Pyridyl | Phenyl | N | N | pol. Rac. | $C_{24}H_{22}N_4O$ (382,2) | 383 $M+H^+$ |

Tabelle 2

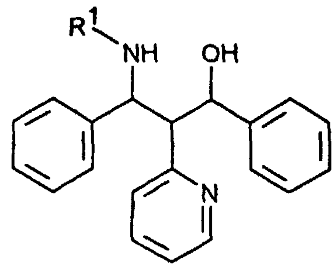

| Beispiel | $R^1$ | | Summenformel (Molmasse) | MS (FAB) |
|---|---|---|---|---|
| 18 | 2-(3-Nitropyridyl) | unp. Rac. | $C_{25}H_{24}N_4O_3$ (426,2) | 427,2 $M+H^+$ |
| 19 | 2-(3-Nitropyridyl) | pol. Rac. | $C_{25}H_{24}N_4O_3$ (426,2) | 427,2 $M+H^+$ |
| 20 | 2-(3-Aminopyridyl) | unp. Rac. | $C_{25}H_{24}N_4O$ (396,2) | 397,3 $M+H^+$ |
| 21 | 2-(3-Aminopyridyl) | pol. Rac. | $C_{25}H_{24}N_4O$ (396,2) | 397,3 $M+H^+$ |
| 22 | 2-(5-Nitropyridyl) | unp. Rac. | $C_{25}H_{22}N_4O_3$ (426,2) | 427,1 $M+H^+$ |
| 23 | 2-(5-Nitropyridyl) | pol. Rac. | $C_{25}H_{22}N_4O_3$ (426,2) | 427,1 $M+H^+$ |
| 24 | 2-(5-Aminopyridyl) | unp. Rac. | $C_{25}H_{24}N_4O$ (396,2) | 397,1 $M+H^+$ |
| 25 | 2-(3-Hydroxypyridyl) | 1. Rac. | $C_{25}H_{23}N_3O_2$ (397,2) | 398 $M+H^+$ |
| 26 | 2-(3-Hydroxypyridyl) | 2. Rac. | $C_{25}H_{23}N_3O_2$ (397,2) | 398 $M+H^+$ |

| Beispiel | $R^1$ | | Summenformel (Molmasse) | MS (FAB) |
|---|---|---|---|---|
| 27 | 2-(3-Hydroxypyridyl) | 3. Rac. | $C_{25}H_{23}N_3O_2$ (397,2) | 398 $M+H^+$ |
| 28 | 2-(3-Benzyloxypyridyl) | 1. Rac. | $C_{32}H_{29}N_3O_2$ (487,3) | 488 $M+H^+$ |
| 29 | 2-(3-Benzyloxypyridyl) | 2. Rac. | $C_{32}H_{29}N_3O_2$ (487,3) | 488 $M+H^+$ |
| 30 | 2-(3-Benzyloxypyridyl) | 3. Rac. | $C_{32}H_{29}N_3O_2$ (487.3) | 488 $M+H^+$ |
| 31 | 2-(5-Methoxypyridyl) | unp. Rac. | $C_{26}H_{25}N_3O_2$ (411,2) | 412 $M+H^+$ |
| 32 | 2-(5-Methoxypyridyl) | pol. Rac. | $C_{26}H_{25}N_3O_2$ (411,2) | 412 $M+H^+$ |
| 33 | 2-(5-Ethoxypyridyl) | 1. Rac. | $C_{27}H_{27}N_3O_2$ (425,2) | 426 $M+H^+$ |
| 34 | 2-(5-Ethoxypyridyl) | 2. Rac. | $C_{27}H_{27}N_3O_2$ (425,2) | 426 $M+H^+$ |
| 35 | 2-(5-Ethoxypyridyl) | 3. Rac. | $C_{27}H_{27}N_3O_2$ (425,2) | 426 $M+H^+$ |
| 36 | 2-(5-Ethoxypyridyl) | 4. Rac. | $C_{27}H_{27}N_3O_2$ (425,2) | 426 $M+H^+$ |
| 37 | 2-(5-Fluorpyridyl) | unp. Rac. | $C_{25}H_{22}FN_3O$ (399,2) | 400 $M+H^+$ |
| 38 | 2-(5-Fluorpyridyl) | pol. Rac. | $C_{25}H_{22}FN_3O$ (399,2) | 400 $M+H^+$ |
| 39 | 2-(5-Chlorpyridyl) | unp. Rac. | $C_{25}H_{22}ClN_3O$ (415,1) | 416 (418) $M+H^+$ |
| 40 | 2-(5-Chlorpyridyl) | pol. Rac. | $C_{25}H_{22}ClN_3O$ (415,1) | 416 (41) $M+H^+$ |
| 41 | 2-(Pyridyl-5-carbon-säuremethylester) | unp. Rac. | $C_{27}H_{25}N_3O_3$ (439,2) | 440,1 $M+H^+$ |
| 42 | 2-(Pyridyl-5-carbon-säuremethylester) | pol. Rac. | $C_{27}H_{25}N_3O_3$ (439,2) | 440,1 $M+H^+$ |

| Beispiel | $R^1$ | | Summenformel (Molmasse) | MS (FAB) |
|---|---|---|---|---|
| 43 | 2-(Pyridyl-5-carbonsäure) | unp. Rac. | $C_{26}H_{23}N_3O_3$ (425,2) | 426,2 $M+H^+$ |
| 44 | 2-(Pyridyl-5-carbonsäure) | pol. Rac. | $C_{26}H_{23}N_3O_3$ (425,2) | 426,2 $M+H^+$ |
| 45 | 2-(Pyridyl-5-carbonsäure) | | $C_{26}H_{24}N_4O_2$ (424,2) | 425 $M+H^+$ |
| 46 | 3-Hydroxyphenyl | unp. Rac. | $C_{26}H_{24}N_2O_2$ (396,2) | 397 $M+H^+$ |
| 47 | 3-Hydroxyphenyl | pol. Rac. | $C_{26}H_{24}N_2O_2$ (396,2) | 397 $M+H^+$ |
| 48 | Phenyl-2-carbonsäuremethylester | unp. Rac. | $C_{28}H_{26}N_2O_3$ (438,2) | 439,2 $M+H^+$ |
| 49 | Phenyl-2-carbonsäuremethylester | pol. Rac. | $C_{28}H_{26}N_2O_3$ (438,2) | 439,2 $M+H^+$ |
| 50 | Phenyl-2-carbonsäure | | $C_{27}H_{24}N_2O_3$ (424,2) | 425,2 $M+H^+$ |

Tabelle 3

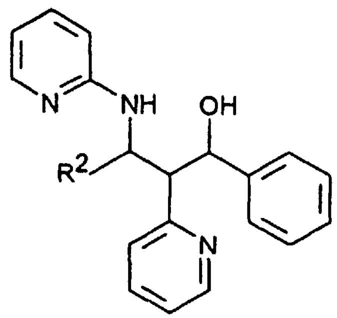

| Beispiel | $R^2$ | | Summenformel (Molmasse) | MS (FAB) |
|---|---|---|---|---|
| 51 | 2-Nitrophenyl | unp. Rac. | $C_{25}H_{22}N_4O_3$ (426,2) | 427,2 $M+H^+$ |
| 52 | 2-Nitrophenyl | pol. Rac. | $C_{25}H_{22}N_4O_3$ (426,2) | 427,2 $M+H^+$ |
| 53 | 3-Nitrophenyl | unp. Rac. | $C_{25}H_{22}N_4O_3$ (426,2) | 427,2 $M+H^+$ |
| 54 | 3-Nitrophenyl | pol. Rac. | $C_{25}H_{22}N_4O_3$ (426,2) | 427,2 $M+H^+$ |
| 55 | 3-Nitrophenyl | (-)-Enantiomer von Bsp. 53 | | |
| 56 | 3-Nitrophenyl | (+)-Enantiomer von Bsp. 53 | | |
| 57 | 4-Nitrophenyl | unp. Rac. | $C_{25}H_{22}N_4O_3$ (426,2) | 427,1 $M+H^+$ |
| 58 | 4-Nitrophenyl | pol. Rac. | $C_{25}H_{22}N_4O_3$ (426,2) | 427,1 $M+H^+$ |

| Beispiel | $R^2$ | | Summenformel (Molmasse) | MS (FAB) |
|---|---|---|---|---|
| 59 | 2-Aminophenyl | unp. Rac. | $C_{25}H_{24}N_4O$ (396,2) | 397,2 $M+H^+$ |
| 60 | 2-Aminophenyl | pol. Rac. | $C_{25}H_{24}N_4O$ (396,2) | 397,2 $M+H^+$ |
| 61 | 2-Aminophenyl | (-)-Enantiomer von Bsp. 59 | | |
| 62 | 2-Aminophenyl | (+)-Enantiomer von Bsp. 59 | | |
| 63 | 3-Aminophenyl | unp. Rac. | $C_{25}H_{24}N_4O$ (396,2) | 397,3 $M+H^+$ |
| 64 | 3-Aminophenyl | pol. Rac. | $C_{25}H_{24}N_4O$ (396,2) | 397,2 $M+H^+$ |
| 65 | 4-Aminophenyl | unp. Rac. | $C_{25}H_{24}N_4O$ (396,2) | 397,3 $M+H^+$ |
| 66 | 4-Aminophenyl | pol. Rac. | $C_{25}H_{24}N_4O$ (396,2) | 397,2 $M+H^+$ |
| 67 | 2-Hydroxyphenyl | unp. Rac. | $C_{25}H_{23}N_3O_2$ (397,2) | 398,2 $M+H^+$ |
| 68 | 2-Hydroxyphenyl | pol. Rac. | $C_{25}H_{23}N_3O_2$ (397,2) | 398 $M+H^+$ |
| 69 | 2-Hydroxyphenyl | (+)-Enantiomer von Bsp. 67 | $C_{25}H_{23}N_3O_2$ (397,2) | 398 $M+H^+$ |
| 70 | 2-Hydroxyphenyl | (-)-Enantiomer von Bsp. 67 | $C_{25}H_{23}N_3O_2$ (397,2) | 398 $M+H^+$ |
| 71 | 3-Hydroxyphenyl | unp. Rac. | $C_{25}H_{23}N_3O_2$ (397,2) | 398 $M+H^+$ |
| 72 | 3-Hydroxyphenyl | pol. Rac. | $C_{25}H_{23}N_3O_2$ (397,2) | 398 $M+H^+$ |
| 73 | 4-Hydroxyphenyl | unp. Rac. | $C_{25}H_{23}N_3O_2$ (397,2) | 398 $M+H^+$ |
| 74 | 4-Hydroxyphenyl | pol. Rac. | $C_{25}H_{23}N_3O_2$ (397,2) | 398 $M+H^+$ |

| Beispiel | $R^2$ | | Summenformel (Molmasse) | MS (FAB) |
|---|---|---|---|---|
| 75 | 2-Benzyloxyphenyl | unp. Rac. | $C_{32}H_{29}N_3O_2$ (487,2) | 488 $M+H^+$ |
| 76 | 2-Benzyloxyphenyl | pol. Rac. | $C_{32}H_{29}N_3O_2$ (487,2) | 488 $M+H^+$ |
| 77 | 4-Acetoxyphenyl | unp. Rac. | $C_{27}H_{25}N_3O_3$ (439,1) | 440 $M+H^+$ |
| 78 | 4-Acetoxyphenyl | pol. Rac. | $C_{27}H_{25}N_3O_3$ (439,1) | 440 $M+H^+$ |

Tabelle 4

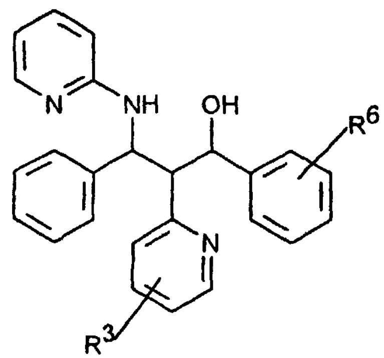

| Beispiel | $R^3$ | $R^6$ | | Summenformel (Molmasse) | MS (FAB) |
|---|---|---|---|---|---|
| 79 | 4-Methoxy | H | unp. Rac. | $C_{26}H_{25}N_3O_2$ (411,2) | 412 $M+H^+$ |
| 80 | 4-Methoxy | H | pol. Rac. | $C_{26}H_{25}N_3O_2$ (411,2) | 412 $M+H^+$ |
| 81 | H | 3-Hydroxy | unp. Rac. | $C_{25}H_{23}N_3O_2$ (397,2) | 398 $M+H^+$ |
| 82 | H | 4-Hydroxy | unp. Rac. | $C_{25}H_{23}N_3O_2$ (397,2) | 398 $M+H^+$ |
| 83 | H | 3-Methoxy | unp. Rac. | $C_{26}H_{25}N_3O_2$ (411,2) | 412 $M+H^+$ |
| 84 | H | 3-Methoxy | pol. Rac. | $C_{26}H_{25}N_3O_2$ (411,2) | 412 $M+H^+$ |
| 85 | H | 4-Methoxy | unp. Rac. | $C_{26}H_{25}N_3O_2$ (411,2) | 412 $M+H^+$ |
| 86 | H | 4-Methoxy | pol. Rac. | $C_{26}H_{25}N_3O_2$ (411,2) | 412 $M+H^+$ |

| Beispiel | $R^3$ | $R^6$ | | Summenformel (Molmasse) | MS (FAB) |
|---|---|---|---|---|---|
| 87 | H | 4-Amino | unp. Rac. | $C_{25}H_{24}N_4O$ (396,2) | 397 $M+H^+$ |
| 88 | H | 4-Amino | pol. Rac. | $C_{25}H_{24}N_4O$ (396,2) | 397 $M+H^+$ |
| 89 | H | 4-Benzyloxycarbonylamido | unp. Rac. | $C_{33}H_{30}N_4O_3$ (530,2) | 531 $M+H^+$ |
| 90 | H | 4-Benzyloxycarbonylamido | pol. Rac. | $C_{33}H_{30}N_4O_3$ (530,2) | 531 $M+H^+$ |

Tabelle 5

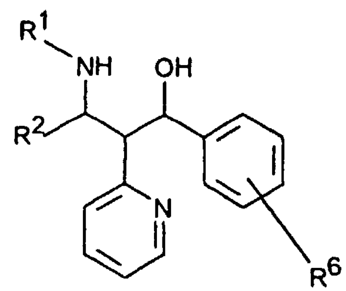

| Beispiel | $R^1$ | $R^2$ | $R^6$ | Summenformel (Molmasse) | MS (FAB) |
|---|---|---|---|---|---|
| 91 | Pyridyl | 2-Aminophenyl | 4-Methoxy | $C_{26}H_{26}N_4O_2$ (426,2) | 427 $M+H^+$ |
| 92 | Pyridyl | 2-Nitro-5-hydroxyphenyl | H | $C_{25}H_{22}N_4O_4$ (442,2) | 443,1 $M+H^+$ |
| 93 | Pyridyl | 2-Amino-5-hydroxyphenyl | H | $C_{25}H_{22}N_4O_4$ (412,2) | 413,2 $M+H^+$ |
| 94 | 3,5-Bis-(trifluor-methyl)-phenyl | Phenyl | H | $C_{28}H_{22}F_6NO$ | |

| Beispiel | Struktur | Summenformel (Molmasse) | MS (FAB) |
|---|---|---|---|
| 95 | 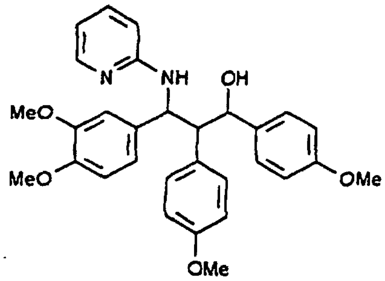 | $C_{30}H_{32}N_2O_5$ (500,2) | 501 M+H$^+$ |

Beispiel 97

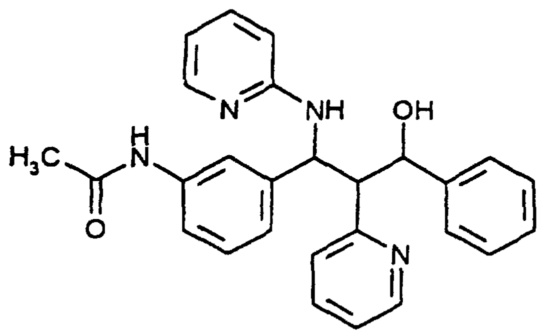

300 mg (0,76 mmol) Aminoverbindung aus Beispiel 63 wurden in 10 ml Pyridin gelöst, mit 75 µl (0,80 mmol) Essigsäureanhydrid und 5 mg Dimethylaminopyridin versetzt und 2 h bei Zimmertemperatur gerührt. Danach wurden 30 ml Wasser zugegeben und 3x mit Essigester extrahiert. Die organischen Phasen wurden getrocknet und eingeengt. Kieselgelchromatographie mit n-Heptan/Essigester 4:1 ergaben 200 mg (60 %) Produkt.
$C_{27}H_{26}N_4O_2$ (438,2) MS (FAB) 439,2 M+H$^+$

Beispiel 98

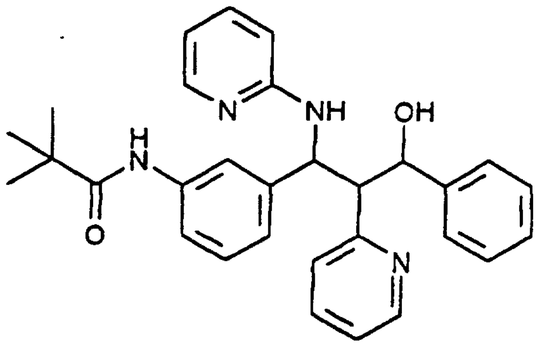

In Analogie zu Beispiel 97 wurde unter Verwendung von Pivalinsäurechlorid die oben angegebene Verbindung erhalten.
$C_{30}H_{32}N_4O_2$ (480,3) MS (FAB) 481,3 $M+H^+$

Beispiel 99

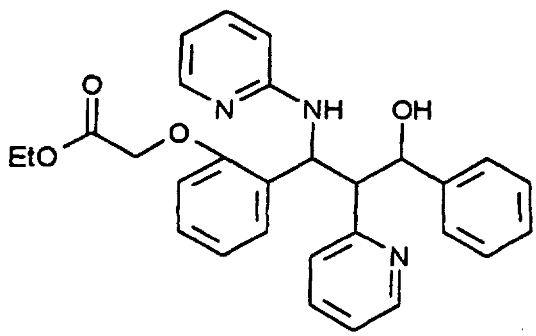

1,99 g (0,005 mol) Beispiel 67 und 1 g gepulvertes Kaliumcarbonat wurden in 50 ml Dimethylformamid vorgelegt. Zur Lösung wurden 0,7 ml (0,006 mol) Bromessigsäureethylester zugegeben und 6 h unter Rückfluß erhitzt. Anschließend wurde im Vakuum eingeengt und der Rückstand über Kieselgel mit n-Heptan/Essigester 2:1 chromatographiert. Ausbeute 1,94 g (80 %)
$C_{29}H_{29}N_3O_4$ (483) MS (FAB) 484 $M+H^+$

Beispiel 100

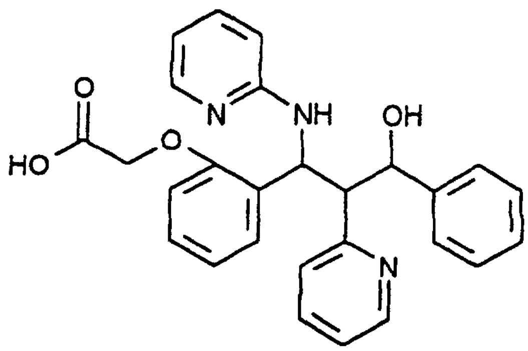

Beispiel 100 wurde aus Beispiel 99 nach dem für Beispiel 4 beschriebenen Verfahren hergestellt.
$C_{27}H_{25}N_3O_4$ (455) MS (FAB) 456 $M+H^+$

Beispiel 101

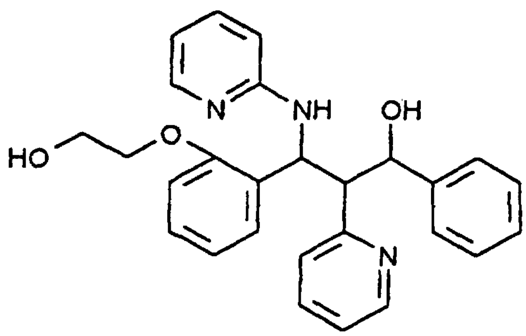

1,99 g (0,005 mol) Beispiel 67 und 8,8 g (0,1 mol) Ethylencarbonat wurden im Ölbad auf 90-95°C erhitzt (Schmelze). Bei dieser Temperatur wurden 0,14 g (0,001 mol) Kaliumcarbonat zugegeben und 5 h gerührt. Nach dem Abkühlen wurde die erhaltene Lösung filtriert und im Vakuum eingeengt. Chromatographie über Kieselgel mit n-Heptan/Essigester 1:1 ergab 1,5 g (68 %) Produkt.
$C_{27}H_{27}N_3O_3$ (441) MS (FAB) 442 $M+H^+$

Beispiel 102

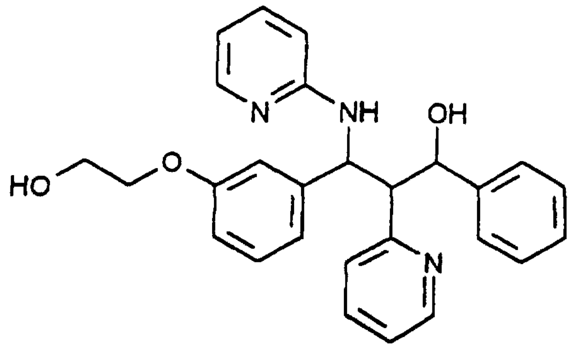

Beispiel 102 wurde aus Beispiel 71 analog zu dem für Beispiel 101 beschriebenen Verfahren erhalten $C_{27}H_{27}N_3O_3$ (441) MS (FAB) 442 $M+H^+$

Beispiel 103

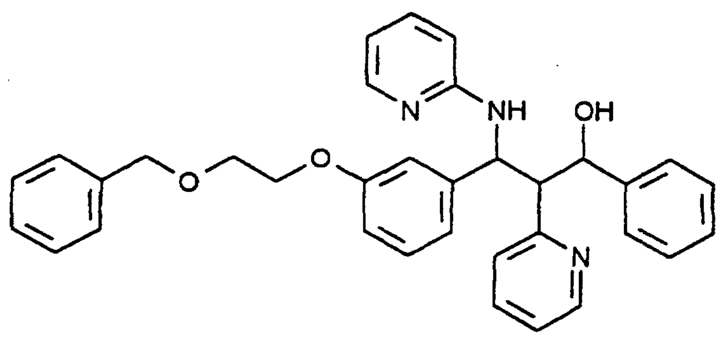

Zu einer Lösung von 1,83 g (12 mmol) Benzyloxyethanol und 3,67 g (14 mmol) Triphenylphosphin in 100 ml trockenem THF wurden unter Argon 4,04 g (20 mmol) Diisopropylazodicarboxylat und danach 3,97 g (10 mmol) Beispiel 71 zugegeben. Nach Rühren über Nacht wurde das Lösungsmittel abgezogen und erneut in Essigester gelöst. Diese Lösung wird 2x mit $Na_2CO_3$-Lösung ausgeschüttelt, dann getrocknet und eingeengt. Kieselgelchromatographie ergab 3,85 g (72 %) Produkt.
$C_{34}H_{33}N_3O_3$ (531,3) MS (FAB) 532 $M+H^+$

| Bsp. | Struktur | Summenformel (Molmasse) Bemerkung | MS |
|---|---|---|---|
| 104 | 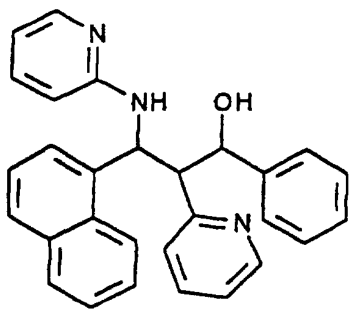 | $C_{29}H_{25}N_3O$ (431,54)<br>unpolares Rac. | 432 (M+1) |
| 105 | 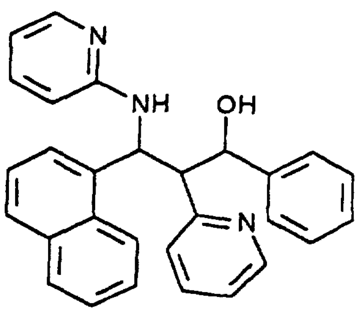 | $C_{29}H_{25}N_3O$ (431,54)<br>polares Rac. | 432 (M+1) |
| 106 | 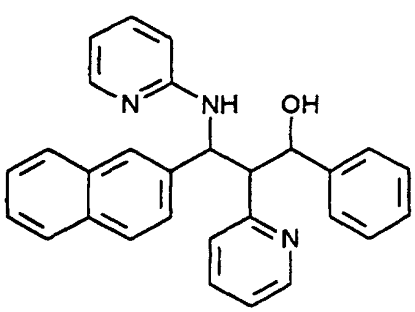 | $C_{29}H_{25}N_3O$ (431,54)<br>polares Rac. | 432 (M+1) |
| 107 | 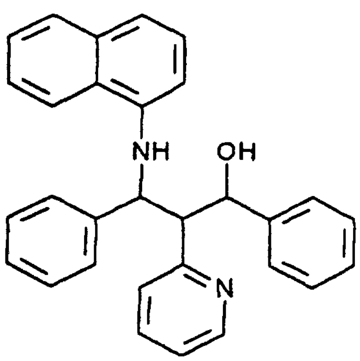 | $C_{30}H_{26}N_2O$ (430,55)<br>unpolares Rac. | 421 (M+1) |

| Bsp. | Struktur | Summenformel (Molmasse) Bemerkung | MS |
|---|---|---|---|
| 108 | 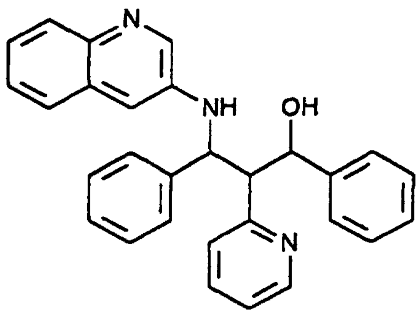 | $C_{29}H_{25}N_3O$ (431,54)<br>unpolares Rac. | 432 (M+1) |
| 109 | 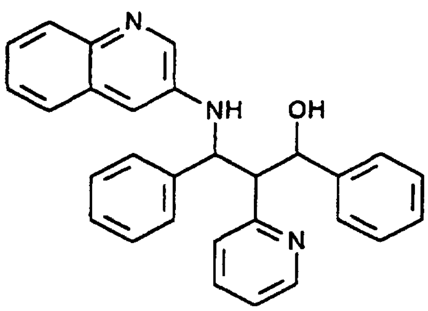 | $C_{29}H_{25}N_3O$ (431,54)<br>mittelpolares Rac. | 432 (M+1) |
| 110 | 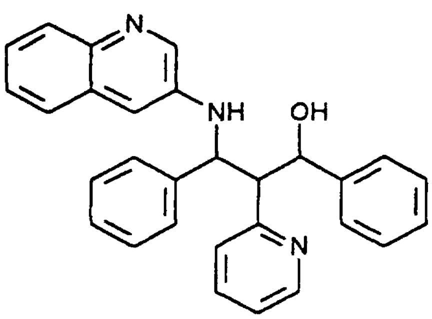 | $C_{29}H_{25}N_3O$ (431,54)<br>polares Rac. | 432 (M+1) |
| 111 | 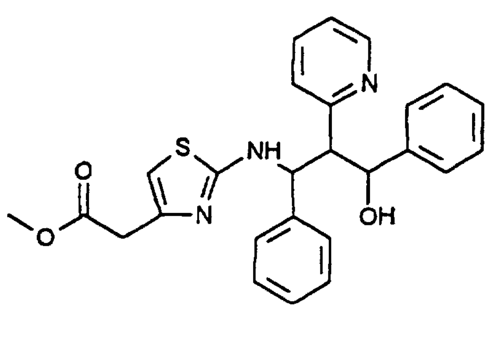 | $C_{26}H_{25}N_3O_3S$ (431,54)<br>polares Rac. | 432 (M+1) |

| Bsp. | Struktur | Summenformel (Molmasse) Bemerkung | MS |
|---|---|---|---|
| 112 | 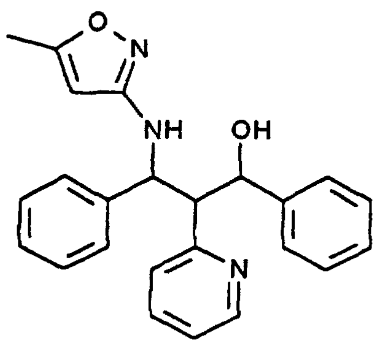 | $C_{24}H_{23}N_3O_2$ (385,47) unpolares Rac. | 386 (M+1) |
| 113 | 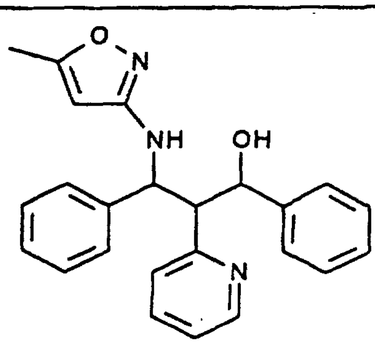 | $C_{24}H_{23}N_3O_2$ (385,47) mittelpolares Rac. | 386 (M+1) |
| 114 | 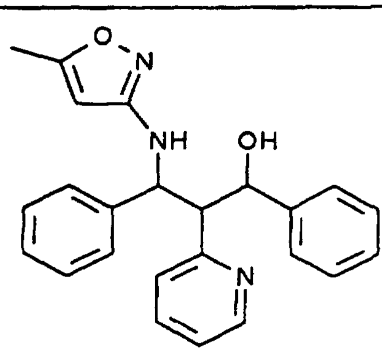 | $C_{24}H_{23}N_3O_2$ (385,47) polares Rac. | 386 (M+1) |
| 115 | 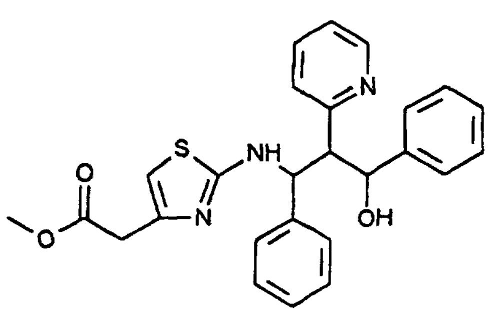 | $C_{26}H_{25}N_3O_3S$ (459,57) unpolares Rac. | 460 (M+1) |

| Bsp. | Struktur | Summenformel (Molmasse) Bemerkung | MS |
|---|---|---|---|
| 116 | 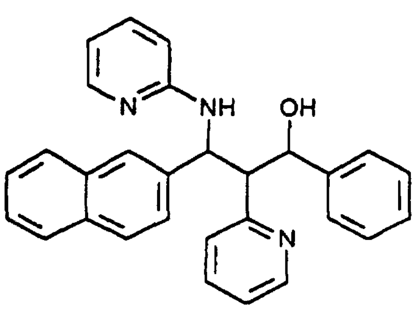 | $C_{29}H_{25}N_3O$ (431,54) unpolares Rac. | 432 (M+1) |
| 117 | 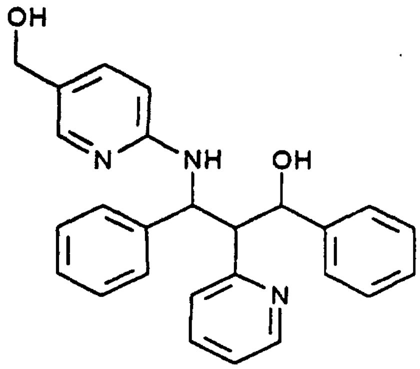 | $C_{26}H_{25}N_3O_2$ (411,51) stärker polares Rac. | 412 (M+H$^+$) |
| 118 | 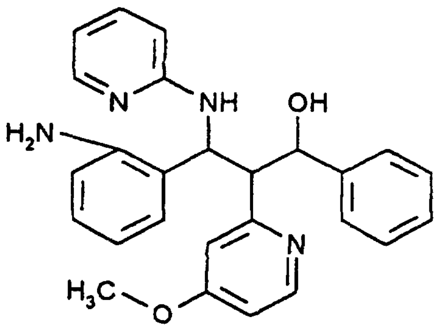 | $C_{26}H_{25}N_4O_2$ (426,52) unpolares Rac. | 427 (M+H$^+$) |

| Bsp. | Struktur | Summenformel (Molmasse) Bemerkung | MS |
|---|---|---|---|
| 119 | 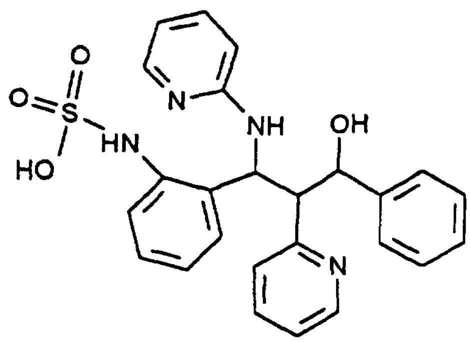 | $C_{25}H_{24}N_4O_4S$ (476,56) Stereoisomeren-mischung | 477 (M+H$^+$) |
| 120 | 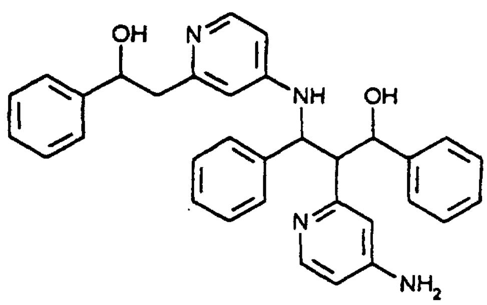 | $C_{33}H_{32}N_4O_2$ (516,65) Stereoisomeren-mischung | 515 (M+H$^+$) |
| 121 | 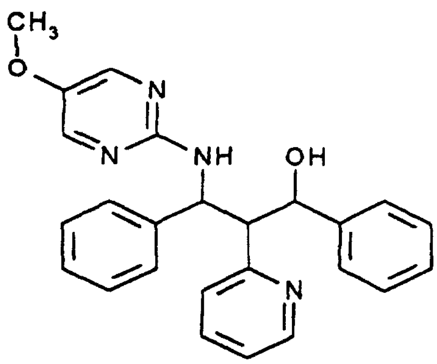 | $C_{25}H_{24}N_4O_2$ (412,5) weniger polares Rac. | 413 (M+H$^+$) |

| Bsp. | Struktur | Summenformel (Molmasse) Bemerkung | MS |
|---|---|---|---|
| 122 | 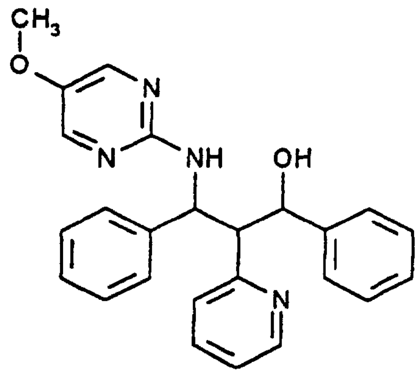 | $C_{25}H_{24}N_4O_2$ (412,5)<br><br>stärkerer polares Rac. | 413 ($M+H^+$) |
| 123 | 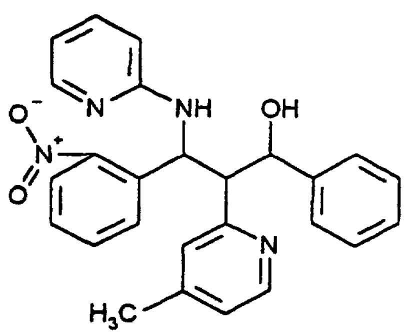 | $C_{26}H_{24}N_4O_3$ (440,51)<br><br>weniger polares Rac. | 441 ($M+H^+$) |
| 124 | 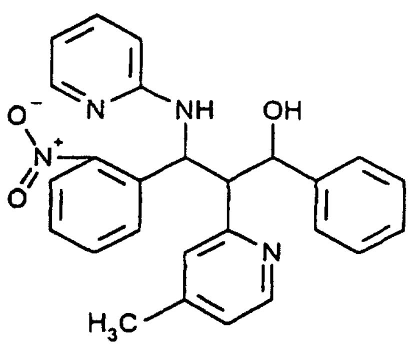 | $C_{26}H_{24}N_4O_3$ (440,51)<br><br>stärker polares Rac. | 441 ($M+H^+$) |

| Bsp. | Struktur | Summenformel (Molmasse) Bemerkung | MS |
|---|---|---|---|
| 125 | 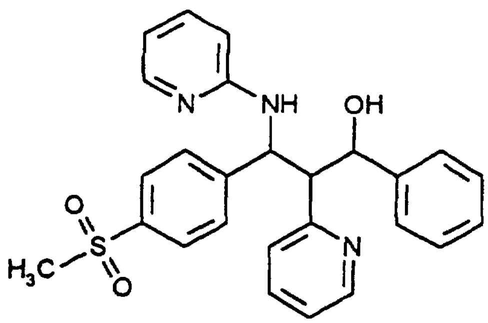 | $C_{26}H_{25}N_3O_3S$ (459,57)<br>weniger polares Rac. | 460 $(M+H^+)$ |
| 126 | 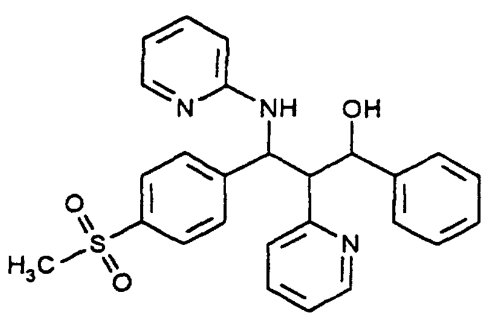 | $C_{26}H_{25}N_3O_3S$ (459,57)<br>stärker polares Rac. | 460 $(M+H^+)$ |
| 127 | 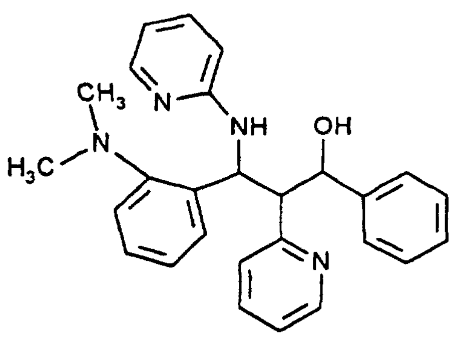 | $C_{27}H_{28}N_4O$ (424,55)<br>weniger polares Rac. | 425 $(M+H^+)$ |

**Patentansprüche**

**1.** Propanolaminderivate der Formel I,

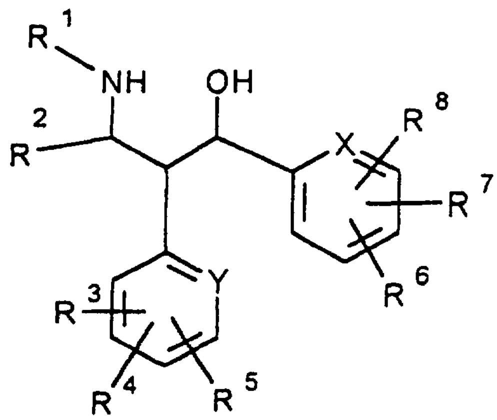

I

worin bedeuten

$R^1$ und $R^2$ unabhängig voneinander Cycloalkyl mit 3-8 Ring-C-Atomen, Phenyl, Naphthyl, Phenanthryl, Pyridyl, Thienyl, Furyl, Pyrimidyl, Indolyl, Thiazolyl, Imidazolyl, Coumarinyl, Phthaliminyl, Chinoyl, Piperazinyl, Tetrazolyl, Triazolyl, Oxazolyl oder deren thieno-, pyridino- oder benzoannelierte Derivate, wobei der Cycloalkylring, Aromat oder Heteroaromat ein bis dreifach substituiert sein kann mit Fluor, Chlor, Brom, Jod, OH, $CF_3$, $-NO_2$, CN, $(C_1-C_8)$-Alkoxy, $(C_1-C_8)$-Alkyl, $NH_2$, -NH-$R^9$, -N($R^9$)$R^{10}$, CHO, -COOH, -$COOR^{11}$, -(C=O)-$R^{12}$, $(C_1-C_6)$-alkyl-OH, $(C_1-C_6)$-alkyl(-OH)-Phenyl, $(C_1-C_6)$-alkyl-$CF_3$, $(C_1-C_6)$-alkyl-$NO_2$, $(C_1-C_6)$-alkyl-CN, $(C_1-C_6)$-alkyl-$NH_2$, $(C_1-C_6)$-alkyl-NH-$R^9$, $(C_1-C_6)$-alkyl-N($R^9$) $R^{10}$, $(C_1-C_6)$-alkyl-CHO, $(C_1-C_6)$-alkyl-COOH, $(C_1-C_6)$-alkyl-$COOR^{11}$, $(C_1-C_6)$-alkyl-(C=O)-$R^{12}$, -O-$(C_1-C_6)$-alkyl-OH, -O-$(C_1-C_6)$-alkyl-$CF_3$, -O-$(C_1-C_6)$-alkyl-$NO_2$, -O-$(C_1-C_6)$-alkyl-CN, -O-$(C_1-C_6)$-alkyl-$NH_2$, -O-$(C_1-C_6)$-alkyl-NH-$R^9$, -O-$(C_1-C_6)$-alkyl-N($R^9$)$R^{10}$, -O-$(C_1-C_6)$-alkyl-CHO, -O-$(C_1-C_6)$-alkyl-COOH, -O-$(C_1-C_6)$-alkyl-$COOR^{11}$, -O-$(C_1-C_6)$-alkyl-(C=O)-$R^{12}$, -N-$SO_3H$, -$SO_2$-$CH_3$, -O-$(C_1-C_6)$-alkyl-O-$(C_1-C_6)$-alkyl-Phenyl, wobei in den Alkylresten ein oder mehrere Wasserstoff(e) durch Fluor ersetzt sein können;

$R^3$ bis $R^8$ unabhängig voneinander Wasserstoff, Fluor, Chlor, Brom, Jod, OH, $CF_3$, $-NO_2$, CN, $(C_1-C_8)$-Alkoxy, $(C_1-C_8)$-Alkyl, $NH_2$, -NH-$R^9$, -N($R^9$)$R^{10}$, CHO, -COOH, -$COOR^{11}$, -(C=O)-$R^{12}$, wobei in den Alkylresten ein oder mehrere Wasserstoff(e) durch Fluor ersetzt sein können;

$R^9$ bis $R^{12}$ unabhängig voneinander Wasserstoff, ($C_1$-$C_8$-Alkyl;

X CH, NH;

Y CH, NH;

mit der Maßgabe, daß die Reste $R^1$, $R^2$, X und Y nicht gleichzeitig die folgende Bedeutung haben

$R^1$ Phenyl, einschließlich substituiertes Phenyl;
$R^2$ Phenyl, einschließlich substituiertes Phenyl;
X CH;
Y CH;

sowie deren physiologisch verträglichen Säureadditionssalze.

2. Verbindungen der Formel I, gemäß Anspruch 1, **dadurch gekennzeichnet, daß** darin bedeuten

$R^1$ und $R^2$ unabhängig voneinander Cycolalkyl mit 3-8 Ring-C-Atomen, Phenyl, Naphthyl, Thienyl, Furyl, Pyrimidyl, Thiazolyl, Imidazolyl, Phthaliminyl, Chinoyl, Piperazinyl, Tetrazolyl, Triazolyl, Oxazolyl oder deren benzoannelierte Derivate, wobei der Cycloalkylring, Aromat oder Heteroaromat ein bis dreifach substituiert sein kann mit Fluor, Chlor, Brom, OH, $CF_3$, $-NO_2$, CN, $(C_1-C_8)$-Alkoxy, $(C_1-C_8)$-Alkyl, $NH_2$, -NH-$R^9$, -N($R^9$)$R^{10}$, -COOH, -$COOR^{11}$, -(C=O)-$R^{12}$, wobei in den Alkylresten ein oder mehrere Wasserstoff(e) durch Fluor ersetzt sein können;

$R^3$ bis $R^8$ unabhängig voneinander Wasserstoff, Fluor, Chlor, Brom, OH, $CF_3$, $-NO_2$, CN, $(C_1-C_8)$-Alkoxy, $(C_1-C_8)$-Alkyl, $NH_2$, $-NH-R^9$, $-N(R^9)R^{10}$, -COOH, $-COOR^{11}$, $-(C=O)-R^{12}$, wobei in den Alkylresten ein oder mehrere Wasserstoff(e) durch Fluor ersetzt sein können;

$R^9$ bis $R^{12}$ unabhängig voneinander Wasserstoff, $(C_1-C_8)$-Alkyl;

X CH, NH;

Y CH, NH;

mit der Maßgabe, daß die Reste $R^1$, $R^2$, X und Y nicht gleichzeitig die folgende Bedeutung haben

$R^1$ Phenyl, einschließlich substituiertes Phenyl;
$R^2$ Phenyl, einschließlich substituiertes Phenyl;
X CH;
Y CH;

sowie deren physiologisch verträglichen Säureadditionssalze.

**3.** Verbindungen der Formel I, gemäß Anspruch 1 oder 2, **dadurch gekennzeichnet, daß** darin bedeuten

$R^1$ Pyridyl, Pyrimidyl, Thienyl, Thiazolyl, wobei der Heteroaromat ein bis dreifach substituiert sein kann mit Fluor, Chlor, Brom, Jod, OH, $CF_3$, $-NO_2$, CN, $(C_1-C_8)$-Alkoxy, $(C_1-C_8)$-Alkyl, $NH_2$, $-NH-R^9$, $-N(R^9)R^{10}$, CHO, -COOH, $-COOR^{11}$, $-(C=O)-R^{12}$;

$R^2$ Phenyl, wobei der Aromat ein bis dreifach substituiert sein kann mit Fluor, Chlor, Brom, OH, $CF_3$, $-NO_2$, CN, $(C_1-C_8)$-Alkoxy, $(C_1-C_8)$-Alkyl, $NH_2$, $-NH-R^9$, $-N(R^9)R^{10}$, -COOH, $-COOR^{11}$, $-(C=O)-R^{12}$;

$R^3$ bis $R^8$ unabhängig voneinander Wasserstoff, Fluor, Chlor, Brom, Jod, OH, $CF_3$, $-NO_2$, CN, $(C_1-C_8)$-Alkoxy, $(C_1-C_8)$-Alkyl, $NH_2$, $-NH-R^9$, $-N(R^9)R^{10}$, CHO, -COOH, $-COOR^{11}$, $-(C=O)-R^{12}$, wobei in den Alkylresten ein oder mehrere Wasserstoff(e) durch Fluor ersetzt sein können;

$R^9$ bis $R^{12}$ unabhängig voneinander Wasserstoff, $(C_1-C_{8)}$-Alkyl;

X CH;

Y NH;

sowie deren physiologisch verträglichen Säureadditionssalze.

**4.** Verfahren zur Herstellung von Verbindungen der Formel I, gemäß einem oder mehreren der Ansprüche 1 bis 3, **dadurch gekennzeichnet, daß** man nach folgendem Formelschema

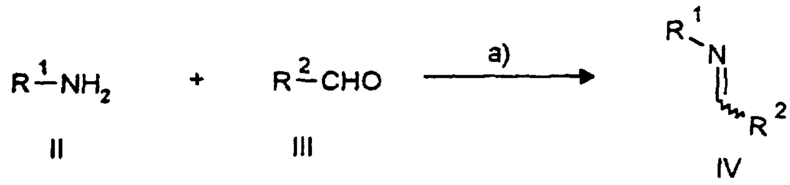


II III IV

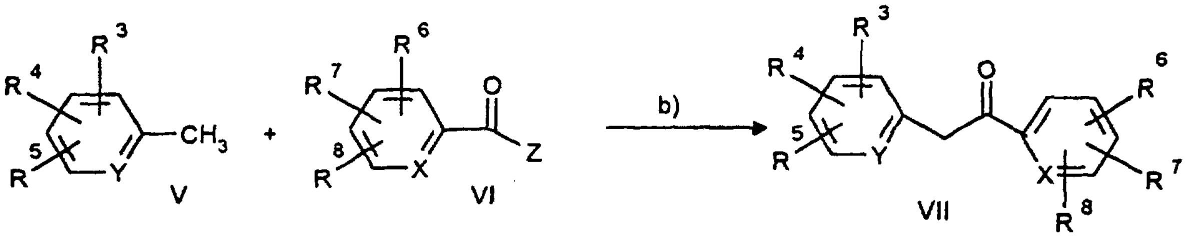


V VI VII

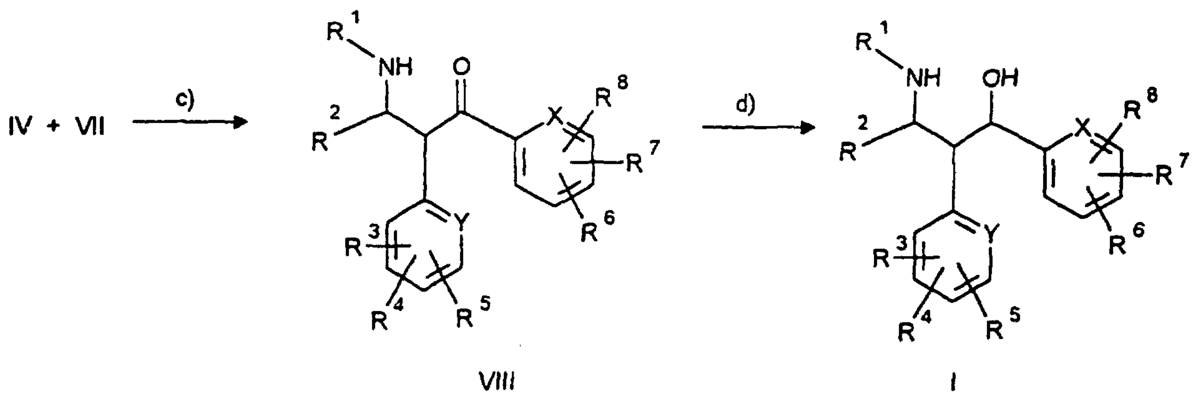


VIII I

a) ein Imin der Formel IV, in der $R^1$ und $R^2$ die zu Formel I angegebene Bedeutung haben, durch Reaktion eines Amins der Formel II mit einem Aldehyd der Formel III herstellt und
b) eine Ketoverbindung der Formel VII in der X, Y und $R^3$ bis $R^8$ die zu Formel I angegebene Bedeutung haben, durch Reaktion einer Verbindung der Formel V mit einer Verbindung der Formel VI herstellt und
c) eine Verbindung der Formel VIII in der X, Y und $R^1$ bis $R^8$ die zu Formel I angegebenen Bedeutungen haben, durch Reaktion einer Verbindung IV mit einer Verbindung der Formel VII herstellt und
d) die Verbindung der Formel VIII in einem geeigneten Lösungsmittel bei einer Temperatur von -30°C bis +40°C mit einem geeigneten Reduktionsmittel zu einer Verbindung der Formel I reduziert.

5. Verfahren zur Herstellung von Verbindungen der Formel I, gemäß einem oder mehreren der Ansprüche 1 bis 3, **dadurch gekennzeichnet, daß** man nach folgendem Formelschema

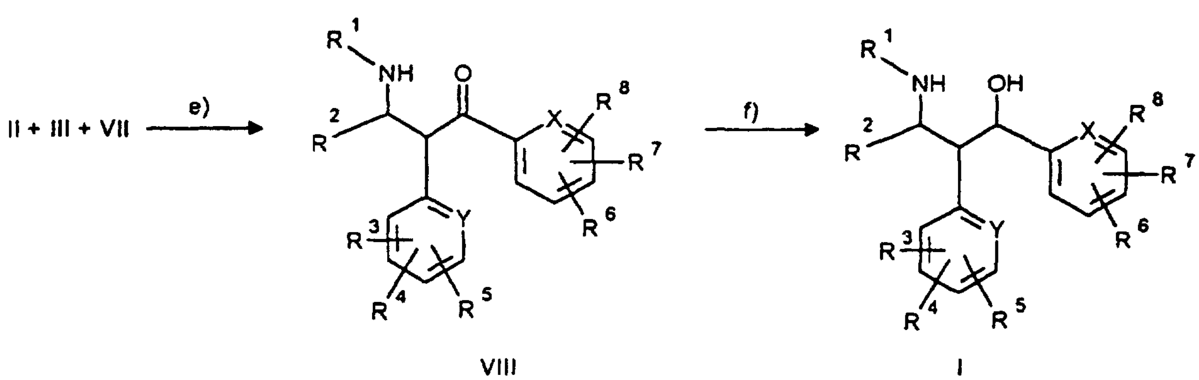


VIII I

e) die Verbindungen der Formeln II, III und VII in einem geeigneten Lösungsmittel bei einer Temperatur von 20°C bis 150°C zu einer Verbindung der Formel VIII umsetzt und
f) die Verbindung der Formel VIII in einem geeigneten Lösungsmittel bei einer Temperatur von -30°C bis +40°C mit einem geeigneten Reduktionsmittel zu einer Verbindung der Formel reduziert.

**6.** Arzneimittel enthaltend eine oder mehrere der Verbindungen gemäß einem oder mehreren der Ansprüche 1 bis 3.

**7.** Arzneimittel enthaltend eine oder mehrere der Verbindungen gemäß einem oder mehreren der Ansprüche 1 bis 3 und ein oder mehrere lipidsenkende Wirkstoffe.

**8.** Verfahren zur Herstellung eines Arzneimittels enthaltend eine oder mehrere der Verbindungen gemäß einem oder mehreren der Ansprüche 1 bis 3, **dadurch gekennzeichnet, daß** der Wirkstoff mit einem pharmazeutisch geeigneten Träger vermischt wird und diese Mischung in eine für die Verabreichung geeignete Form gebracht wird.

**9.** Verwendung der Verbindungen gemäß einem oder mehreren der Ansprüche 1 bis 3 zur Herstellung eines Medikaments zur Behandlung von Hyperlipidämie.

**10.** Verwendung der Verbindungen gemäß einem oder mehreren der Ansprüche 1 bis 3 zur Herstellung eines Medikaments zur Beeinflussung des Serumcholesterinspiegels.

**11.** Verwendung der Verbindungen gemäß einem oder mehreren der Ansprüche 1 bis 3 zur Herstellung eines Medikaments zur Prävention arteriosklerotischer Erscheinungen.

## Claims

**1.** A propanolamine derivative of the formula I,

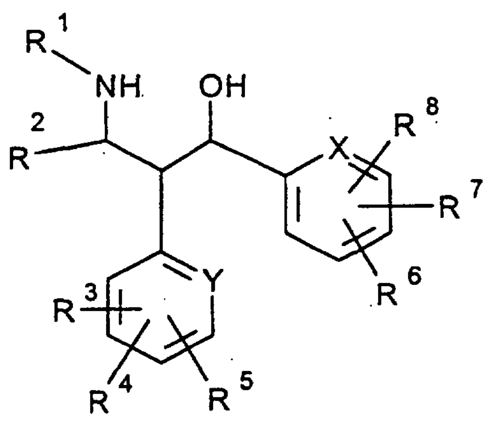

in which

$R^1$ and $R^2$ independently of one another are cycloalkyl having 3-8 ring carbon atoms, phenyl, naphthyl, phenanthryl, pyridyl, thienyl, furyl, pyrimidyl, indolyl, thiazolyl, imidazolyl, coumarinyl, phthalimidyl, quinolyl, piperazinyl, tetrazolyl, triazolyl, oxazolyl or their thieno-, pyridino- or benzo-fused derivatives, it being possible for the cycloalkyl ring, aromatic or heteroaromatic to be mono- to trisubstituted by fluorine, chlorine, bromine, iodine, OH, $CF_3$, $-NO_2$, CN, $(C_1-C_8)$-alkoxy, $(C_1-C_8)$-alkyl, $NH_2$, $-NH-R^9$, $-N(R^9)R^{10}$, CHO, -COOH, $-COOR^{11}$, $-(C{=}O)-R^{12}$, $(C_1-C_6)$-alkyl-OH, $(C_1-C_6)$-alkyl(-OH)-phenyl, $(C_1-C_6)$-alkyl-$CF_3$, $(C_1-C_6)$-alkyl-$NO_2$, $(C_1-C_6)$-alkyl-CN, $(C_1-C_6)$-alkyl-$NH_2$, $(C_1-C_6)$-alkyl-NH-$R^9$, $(C_1-C_6)$-alkyl-$N(R^9)R^{10}$, $(C_1-C_6)$-alky)-CHO, $(C_1-C_6)$-alkyl-COOH, $(C_1-C_6)$-alkyl-$COOR^{11}$, $(C_1-C_6)$-alkyl-$(C{=}O)-R^{12}$, -O-$(C_1-C_6)$-alkyl-OH, -O-$(C_1-C_6)$-alkyl-$CF_3$, -O-$(C_1-C_6)$-alky)-$NO_2$, -O-$(C_1-C_6)$-alkyl-CN, -O-$(C_1-C_6)$-alkyl-$NH_2$, -O-$(C_1-C_6)$-alkyl-NH-$R^9$, -O-$(C_1-C_6)$-alkyl-$N(R^9)R^{10}$, -O-$(C_1-C_6)$-alkyl-CHO, -O-$(C_1-C_6)$-a)kyl-COOH, -O-$(C_1-C_6)$-alkyl-$COOR^{11}$, -O-$(C_1-C_6)$-alkyl-$(C{=}O)-R^{12}$, -N-$SO_3H$, $-SO_2-CH_3$, -O-$(C_1-C_6)$-alkyl-O-$(C_1-C_6)$-alkylphenyl, it being possible in the alkyl radicals

for one or more hydrogen(s) to be replaced by fluorine;

$R^3$ to $R^8$ independently of one another are hydrogen, fluorine, chlorine, bromine, iodine, OH, $CF_3$, $-NO_2$, CN, $(C_1-C_8)$-alkoxy, $(C_1-C_8)$-alkyl, $NH_2$, $-NH-R^9$, $-N(R^9)R^{10}$, CHO, -COOH, $-COOR^{11}$, $-(C=O)-R^{12}$, it being possible in the alkyl radicals for one or more hydrogen(s) to be replaced by fluorine;

$R^9$ to $R^{12}$ independently of one another are hydrogen, $(C_1-C_8)$-alkyl;

X is CH, NH;

Y is CH, NH;

with the proviso that the radicals $R^1$, $R^2$, X and Y do not simultaneously have the following meaning

$R^1$ is phenyl, including substituted phenyl;
$R^2$ is phenyl, including substituted phenyl;
X is CH;
Y is CH;

or its physiologically tolerable acid addition salts.

**2.** A compound of the formula I as claimed in claim 1, wherein

$R^1$ and $R^2$ independently of one another are cycloalkyl having 3-8 ring carbon atoms, phenyl, naphthyl, thienyl, furyl, pyrimidyl, thiazolyl, imidazolyl, phthalimidyl, quinolyl, piperazinyl, tetrazolyl, triazolyl, oxazolyl or their benzo-fused derivatives, it being possible for the cycloalkyl ring, aromatic or heteroaromatic to be mono- to trisubstituted by fluorine, chlorine, bromine, OH, $CF_3$, $-NO_2$, CN, $(C_1-C_8)$-alkoxy, $(C_1-C_8)$-alkyl, $NH_2$, $-NH-R^9$, $-N(R^9)R^{10}$, -COOH, $-COOR^{11}$, $-(C=O)-R^{12}$, it being possible in the alkyl radicals for one or more hydrogen(s) to be replaced by fluorine;

$R^3$ to $R^8$ independently of one another are hydrogen, fluorine, chlorine, bromine, OH, $CF_3$, $-NO_2$, CN, $(C_1-C_8)$-alkoxy, $(C_1-C_8)$-alkyl, $NH_2$, $-NH-R^9$, $-N(R^9)R^{10}$, -COOH, $-COOR^{11}$, $-(C=O)-R^{12}$, it being possible in the alkyl radicals for one or more hydrogen(s) to be replaced by fluorine;

$R^9$ to $R^{12}$ independently of one another are hydrogen, $(C_1-C_8)$-alkyl;

X is CH, NH;

Y is CH, NH;

with the proviso that the radicals $R^1$, $R^2$, X and Y do not simultaneously have the following meaning

$R^1$ is phenyl, including substituted phenyl;
$R^2$ is phenyl, including substituted phenyl;
X is CH;
Y is CH;

or its physiologically tolerable acid addition salts.

**3.** A compound of the formula I as claimed in claim 1 or 2, wherein

$R^1$ is pyridyl, pyrimidyl, thienyl, thiazolyl, it being possible for the heteroaromatic to be mono- to trisubstituted by fluorine, chlorine, bromine, iodine, OH, $CF_3$, $-NO_2$, CN, $(C_1-C_8)$-alkoxy, $(C_1-C_8)$-alkyl, $NH_2$, $-NH-R^9$, $-N(R^9)R^{10}$, CHO, -COOH, $-COOR^{11}$, $-(C=O)-R^{12}$;

$R^2$ is phenyl, it being possible for the aromatic to be mono- to trisubstituted by fluorine, chlorine, bromine, OH, $CF_3$, $-NO_2$, CN, $(C_1-C_8)$-alkoxy, $(C_1-C_8)$-alkyl, $NH_2$, $-NH-R^9$, $-N(R^9)R^{10}$, -COOH, $-COOR^{11}$, $-(C=O)-R^{12}$;

$R^3$ to $R^8$ independently of one another are hydrogen, fluorine, chlorine, bromine, iodine, OH, $CF_3$, $-NO_2$, CN, $(C_1\text{-}C_8)$-alkoxy, $(C_1\text{-}C_8)$-alkyl, $NH_2$, $-NH\text{-}R^9$, $-N(R^9)R^{10}$, CHO, -COOH, $-COOR^{11}$, $-(C{=}O)\text{-}R^{12}$, it being possible in the alkyl radicals for one or more hydrogen(s) to be replaced by fluorine;

$R^9$ to $R^{12}$ independently of one another are hydrogen, $(C_1\text{-}C_8)$-alkyl;

X is CH;

Y is NH;

or its physiologically tolerable acid addition salts.

**4.** A process for the preparation of compounds of the formula I as claimed in one or more of claims 1 to 3, which comprises, according to the following reaction scheme

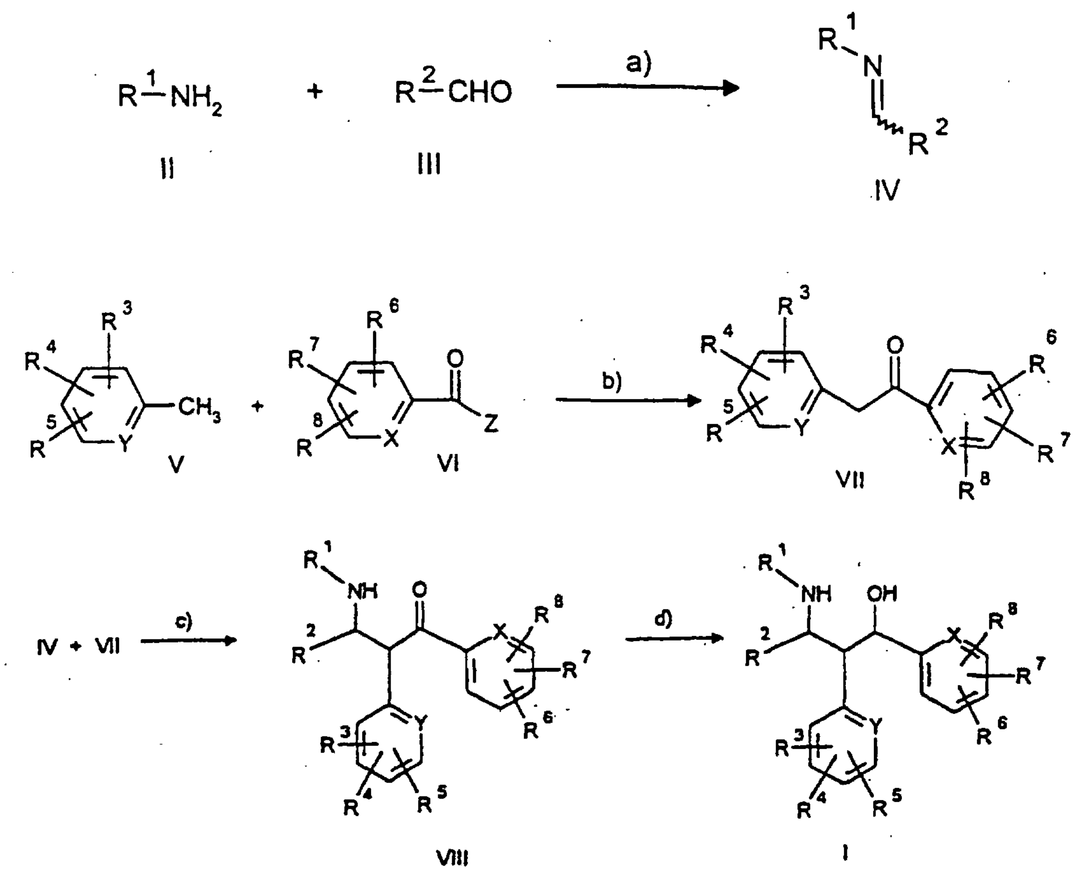


a) preparing an imine of the formula IV, in which $R^1$ and $R^2$ have the meaning indicated for formula I, by reaction of an amine of the formula II with an aldehyde of the formula III and
b) preparing a keto compound of the formula VII in which X, Y and $R^3$ to $R^8$ have the meaning indicated for formula I, by reaction of a compound of the formula V with a compound of the formula VI and
c) preparing a compound of the formula VIII in which X, Y and $R^1$ to $R^8$ have the meanings indicated for formula I, by reaction of a compound IV with a compound of the formula VII and
d) reducing the compound of the formula VIII in a suitable solvent at a temperature from -30°C to +40°C using a suitable reductant to give a compound of the formula I.

**5.** A process for the preparation of compounds of the formula I, as claimed in one or more of claims 1 to 3, which comprises, according to the following reaction scheme

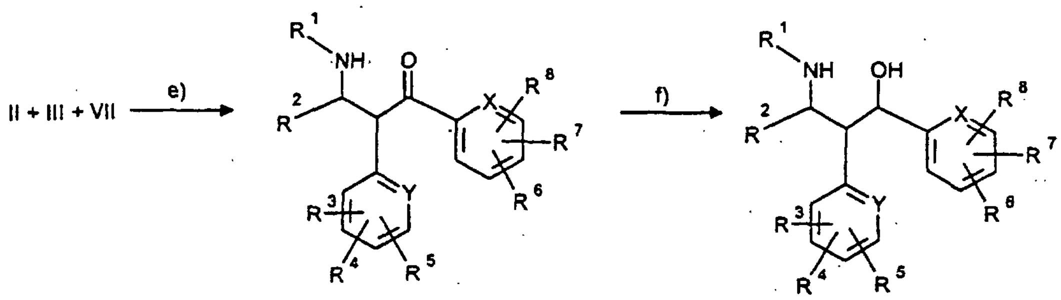


VIII I

e) reacting the compounds of the formulae II, III and VII in a suitable solvent at a temperature from 20°C to 150°C to give a compound of the formula VIII and
f) reducing the compound of the formula VIII in a suitable solvent at a temperature from -30°C to +40°C using a suitable reductant to give a compound of the formula I.

**6.** A pharmaceutical comprising one or more of the compounds as claimed in one or more of claims 1 to 3.

**7.** A pharmaceutical comprising one or more of the compounds as claimed in one or more of claims 1 to 3 and one or more hypolipidemic active compounds.

**8.** A process for the production of a pharmaceutical comprising one or more of the compounds as claimed in one or more of claims 1 to 3, which comprises mixing the active compound with a pharmaceutically suitable carrier and bringing this mixture into a form suitable for administration.

**9.** The use of a compound as claimed in one or more of claims 1 to 3 for the production of a medicament for the treatment of hyperlipidemia.

**10.** The use of a compound as claimed in one or more of claims 1 to 3 for the production of a medicament for affecting the serum cholesterol level.

**11.** The use of a compound as claimed in one or more of claims 1 to 3 for the production of a medicament for the prevention of arteriosclerotic symptoms.

**Revendications**

**1.** Dérivés de propanolamine de formule I

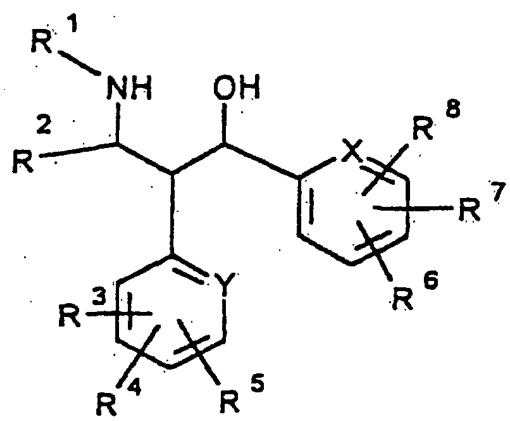

I

$R^1$ et $R^2$, indépendamment l'un de l'autre, représentent un groupe cycloalkyle présentant 3 à 8 atomes de carbone nucléaires, phényle, naphtyle, phénanthryle, pyridyle, thiényle, furyle, pyrimidyle, indolyle,

thiazolyle, imidazolyle, coumarinyle, phtaliminyle, quinoyle, pipérazinyle, tétrazolyle, triazolyle, oxazolyle ou leurs dérivés condensés sur des noyaux thiéno, pyridino ou benzo, le cycle cycloalkyle, l'aromatique ou l'hétéroaromatique peuvent être substitués une à trois fois par un substituant fluor, chlore, brome, iode, OH, $CF_3$, $-NO_2$, CN, alkoxy en $C_1$-$C_8$, alkyle en $C_1$-$C_8$, $NH_2$, $-NH-R^9$, $-N(R^9)R^{10}$, CHO, -COOH, $-COOR^{11}$, $-(C{=}O)-R^{12}$, (alkyl en $C_1$-$C_6$)-OH, (alkyl en $C_1$-$C_6$) (-OH) -phényle, (alkyl en $C_1$-$C_6$) $-CF_3$, (alkyl en $C_1$-$C_6$) $-NO_2$, (alkyl en $C_1$-$C_6$)-CN, (alkyl en $C_1$-$C_6$)-$NH_2$, (alkyl en $C_1$-$C_6$) $-NH-R^9$, (alkyl en $C_1$-$C_6$)-$N(R^9)R^{10}$, (alkyl en $C_1$-$C_6$)-CHO, (alkyl en $C_1$-$C_6$)-COOH, (alkyl en $C_1$-$C_6$) $-COOR^{11}$, (alkyl en $C_1$-$C_6$)-$(C{=}O)-R^{12}$, -O-(alkyl en $C_1$-$C_6$)-OH, -O-(alkyl en $C_1$-$C_6$)-$CF_3$, -O-(alkyl en $C_1$-$C_6$)-$NO_2$, -O-(alkyl en $C_1$-$C_6$)-CN, -O-(alkyl en $C_1$-$C_6$)-$NH_2$, -O-(alkyl en $C_1$-$C_6$)-$NH-R^9$, -O-(alkyl en $C_1$-$C_6$)-$N(R^9)R^{10}$, -O-(alkyl en $C_1$-$C_6$)-CHO, -O-(alkyl en $C_1$-$C_6$)-COOH, -O-(alkyl en $C_1$-$C_6$) $-COOR^{11}$, -O-(alkyl en $C_1$-$C_6$)-$(C{=}O)-R^{12}$, $-N-SO_3H$, $-SO_2-CH_3$, -O-(alkyl en $C_1$-$C_6$)-O-(alkyl en $C_1$-$C_6$)-phényle, un ou plusieurs atomes d'hydrogène dans les restes alkyle peuvent être remplacés par du fluor ;

$R^3$ à $R^8$, indépendamment l'un de l'autre représentent un atome d'hydrogène, de fluor, de chlore, de brome, d'iode, un groupe OH, $CF_3$, $-NO_2$, CN, alkoxy en $C_1$-$C_8$, alkyle en $C_1$-$C_8$, $NH_2$, $-NH-R^9$, $-N(R^9)R^{10}$, CHO, -COOH, $-COOR^{11}$, $-(C{=}O)-R^{12}$, un ou plusieurs atomes d'hydrogène dans les restes alkyle peuvent être remplacés par du fluor ;

$R^9$ à $R^{12}$, indépendamment l'un de l'autre, représentent un atome d'hydrogène, un groupe alkyle en $C_1$-$C_8$ ;

X représente un groupe CH, NH ;

Y représente un groupe CH, NH ;

à condition que les restes $R^1$, $R^2$, X et Y ne possèdent pas en même temps la signification suivante

$R^1$ un groupe phényle, y compris phényle substitué ;
$R^2$ un groupe phényle, y compris phényle substitué ;
X un groupe CH ;
Y un groupe CH ;

ainsi que leurs sels d'addition d'acide physiologiquement tolérés.

2. Composés de formule I, selon la revendication 1, **caractérisés en ce que**

$R^1$ et $R^2$, indépendamment l'un de l'autre, représentent un groupe cycloalkyle présentant 3 à 8 atomes de carbone nucléaires, phényle, naphtyle, thiényle, furyle, pyrimidyle, thiazolyle, imidazolyle, phtaliminyle, quinoyle, pipérazinyle, tétrazolyle, triazolyle, oxazolyle ou leurs dérivés condensés sur des noyaux benzo, le cycle cycloalkyle, l'aromatique ou l'hétéroaromatique peuvent être substitués une à trois fois par un substituant fluor, chlore, brome, OH, $CF_3$, $-NO_2$, CN, alkoxy en $C_1$-$C_8$, alkyle en $C_1$-$C_8$, $NH_2$, $-NH-R^9$, $-N(R^9)R^{10}$, -COOH, $-COOR^{11}$. $-(C{=}O)-R^{12}$, un ou plusieurs atomes d'hydrogène dans les restes alkyle peuvent être remplacés par du fluor ;

$R^3$ à $R^8$, indépendamment l'un de l'autre, représentent un atome d'hydrogène, de fluor, de chlore, de brome, un groupe OH, $CF_3$, $-NO_2$, CN, alkoxy en $C_1$-$C_8$, alkyle en $C_1$-$C_8$, $NH_2$, $-NH-R^9$, $-N(R^9)R^{10}$, -COOH, $-COOR^{11}$, $-(C{=}O)-R^{12}$, un ou plusieurs atomes d'hydrogène dans les restes alkyle peuvent être remplacés par du fluor ;

$R^9$ à $R^{12}$, indépendamment l'un de l'autre, représentent un atome d'hydrogène, un groupe alkyle en $C_1$-$C_8$ ;

X représente un groupe CH, NH ;

Y représente un groupe CH, NH ;

à condition que les restes $R^1$, $R^2$, X et Y ne possèdent pas en même temps la signification suivante

$R^1$ un groupe phényle, y compris phényle substitué ;
$R^2$ un groupe phényle, y compris phényle substitué ;
X un groupe CH ;
Y un groupe CH ;

ainsi que leurs sels d'addition d'acide, physiologiquement tolérés.

3. Composés de formule I, selon la revendication 1 ou 2, **caractérisés en ce que**

$R^1$ représente un groupe pyridyle, pyrimidyle, thiényle, thiazolyle, l'hétéroaromatique peut être substitué une à trois fois par un substituant fluor, chlore, brome, iode, OH, $CF_3$, $-NO_2$, CN, alkoxy en $C_1$-$C_8$, alkyle en $C_1$-$C_8$, $NH_2$, $-NH-R^9$, $-N(R^9)R^{10}$, CHO, -COOH, $-COOR^{11}$, $-(C=O)-R^{12}$,

$R^2$ représente un groupe phényle, l'aromatique peut être substitué une à trois fois par un substituant fluor, chlore, brome, OH, $CF_3$, $-NO_2$, CN, alkoxy en $C_1$-$C_8$, alkyle en $C_1$-$C_8$, $NH_2$, $-NH-R^9$, $-N(R^9)R^{10}$, -COOH, $-COOR^{11}$, $-(C=O)-R^{12}$,

$R^3$ à $R^8$, indépendamment l'un de l'autre, représentent un atome d'hydrogène, de fluor, de chlore, de brome, d'iode, un groupe OH, $CF_3$, $-NO_2$, CN, alkoxy en $C_1$-$C_8$, alkyle en $C_1$-$C_8$, $NH_2$, $-NH-R^9$, $-N(R^9)R^{10}$, CHO, -COOH, $-COOR^{11}$, $-(C=O)-R^{12}$, un ou plusieurs atomes d'hydrogène dans les restes alkyle peuvent être remplacés par du fluor ;

$R^9$ à $R^{12}$, indépendamment l'un de l'autre, représentent un atome d'hydrogène, un groupe alkyle en $C_1$-$C_8$ ;

X représente un groupe CH ;

Y représente un groupe NH ;

ainsi que leurs sels d'addition d'acide physiologiquement tolérés.

**4.** Procédé pour la préparation de composés de formule I, selon une ou plusieurs des revendications 1 à 3, **caractérisé en ce qu'**on opère selon le schéma de formules suivant

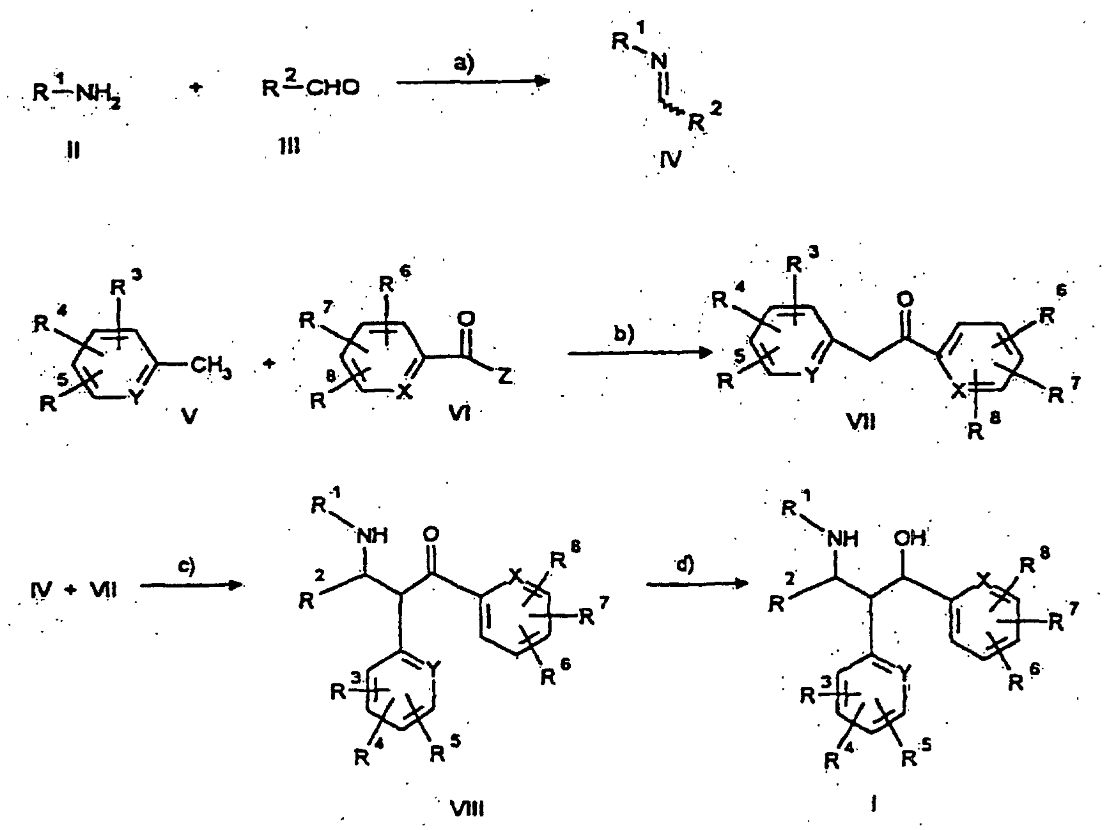


a) préparation d'une imine de formule IV, dans laquelle $R^1$ et $R^2$ possèdent les significations données à la formule I, par réaction d'une amine de formule II sur un aldéhyde de formule III et

b) préparation d'un composé cétonique de formule VII dans laquelle X, Y et $R^3$ à $R^8$ possèdent la signification donnée à la formule I, par réaction d'un composé de formule V sur un composé de formule VI et

c) préparation d'un composé de formule VIII, dans laquelle X, Y et $R^1$ à $R^8$ possèdent la signification donnée à la formule I, par réaction d'un composé IV sur un composé de formulé VII et

d) réduction du composé de formule VIII dans un solvant approprié, à une température de -30°C à +40°C, par un agent réducteur approprié, en un composé de formule 1.

**5.** Procédé pour la préparation de composés de formule I, selon une ou plusieurs des revendications 1 à 3, **caractérisé en ce qu'**on opère selon le schéma de formules suivant

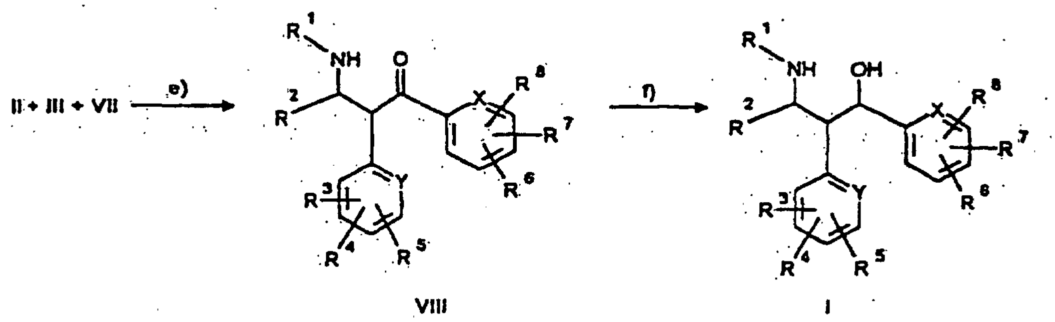


e) transformation des composés de formules II, III et VII dans un solvant approprié, à une température de 20°C à 150°C, en un composé de formule VIII et
f) réduction du composé de formule VIII dans un solvant approprié, à une température de -30°C à +40°C, par un agent réducteur approprié, en un composé de formule I.

**6.** Médicament renfermant un ou plusieurs des composés selon une ou plusieurs des revendications 1 à 3.

**7.** Médicament renfermant un ou plusieurs des composés selon une ou plusieurs des revendications 1 à 3 et un ou plusieurs principes actifs hypolimidémiants.

**8.** Procédé pour la préparation d' un médicament renfermant un ou plusieurs des composés selon une ou plusieurs des revendications 1 à 3, **caractérisé en ce qu'**on mélange le principe actif avec un véhicule approprié en pharmacie et on met ce mélange en forme d'administration appropriée.

**9.** Utilisation des composés selon une ou plusieurs des revendications 1 à 3, pour la préparation d'un médicament pour le traitement d'hyperlipidémie.

**10.** Utilisation des composés selon une ou plusieurs des revendications 1 à 3, pour la préparation d'un médicament en vue d'agir sur le niveau de cholestérol sérique.

**11.** Utilisation des composés selon une ou plusieurs des revendications 1 à 3, pour la préparation d'un médicament pour la prévention de l'apparition de symptômes artériosclérotiques.